# EUROPEAN PATENT APPLICATION

(11) **EP 2 020 405 A1**
(43) Date of publication of application: **04.02.2009**
(21) Application number: 07014946.3
(22) Date of filing: 30.07.2007
(51) Int. Cl.: C07D 211/22, C07D 211/26, C07D 401/06, A61K 31/454, A61K 31/4523, A61P 35/00, A61P 25/24, A61P 3/00

(54) **Substituted aryl or heteroarylpiperidine derivatives as melanocortin-4 receptor modulators**

(71) Applicant: Santhera Pharmaceuticals (Schweiz) AG, 4410 Liestal (CH)
(72) Inventor: Hoffmann-Enger, Barbara, 4414 Füllinsdorf (CH); Lescop, Cyrille, 68680 Kembs (FR); Soeberdt, Michael, 79618 Rheinfelden (DE); Feurer, Achim, 79424 Auggen (DE); Weyermann, Philippp, 4450 Sissach (CH); Nordhoff, Sonja, 4144 Arlesheim (CH); Von Sprecher, Andreas, 4104 Oberwill (CH); Deppe, Holger, 4052 Basel (CH); Bulat, Stephan, 79541 Lörrach (DE)
(74) Representative: Grünecker, Kinkeldey, Stockmair & Schwanhäusser Anwaltssozietät

(57) **Abstract**

The present invention relates to substituted aryl or heteroarylpiperidine derivatives of structure (I) as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

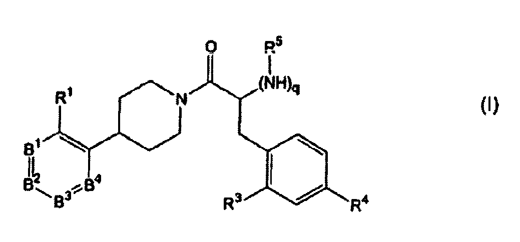

## Description

### Field of the Invention

The present invention relates to substituted aryl or heteroarylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified and these are expressed in different tissues.

MC-1R was first found in melanocytes. Naturally occurring inactive variants of MC-1R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in humans.
The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1 R. The mechanism by which agonism of MC-1 R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti (A^{vy}) mouse which ectopically expresses an antagonist of the MC-1 R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1 R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, (1998)). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (M.J. Owen and C.B. Nemeroff, "Physiology and pharmacology of corticotrophin releasing factor." Pharmacol. Rev. 43: 425-473 (1991)). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Shigeyuki Chaki et al, "Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor", J. Pharm. Exp. Ther. 304(2), 818-826 (2003)).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (D.L. Marks et al. "Role of the central melanocortin system in cachexia." Cancer Res. 61: 1432-1438 (2001)).

Modulators of the melanocortin receptor are already known from the literature. WO 2002/059117 A1 describes piperazine and piperidine derivatives which are claimed to be useful as melanocortin receptor agonists. However, pharmacological data demonstrating the usefulness of the compounds as melanocortin receptor agonists are not disclosed in the application.

WO 2004/024720 A1 describes piperazine urea derivatives which are selective agonists of the human melanocortin-4 receptor and as such they are claimed to be useful in the treatment of prevention of obesity-related disorders.

WO 2005/047253 A1 describes 4,4-disubstituted piperidine derivatives which are postulated to function as melanocortin receptor agonists.

Substituted piperidine derivatives are also described in DE 103 00973 which relates to carboxylic acids and esters having a piperidine ring or a piperazine ring as the central core of the molecule and wherein the core is further substituted in the para-position by a 5-7-membered heterocycle, a phenyl ring, a pyridine ring or a thiazole ring. Said rings are optionally substituted by an ester group. The compounds are used in the preparation of a medicament for the treatment of headaches, non-insulin dependent diabetes mellitus (NIDDM), cardiovascularic diseases, morphintolerance, diseases of the skin, inflammations, allergic rhinitis, asthma, diseases with vascular dilatation and, consequently, with reduced blood circulation in tissues, acute or preemptive treatment of menopausal hot flashes of women with an estrogen deficiency or for the treatment of pain.

In view of the unresolved deficiencies in treatment of various diseases and disorders as discussed above, it is an object of the present invention to provide novel substituted piperidine derivatives with improved ability to cross the blood brain barrier, which are useful as melanocortin-4 receptor modulators to treat cancer cachexia, muscle wasting, anorexia, anxiety, depression, obesity, diabetes, sexual dysfunction and other diseases with MC-4R involvement.

### Summary of the Invention

The present invention relates to substituted aryl or heteroarylpiperidine derivatives of structural formula (I) 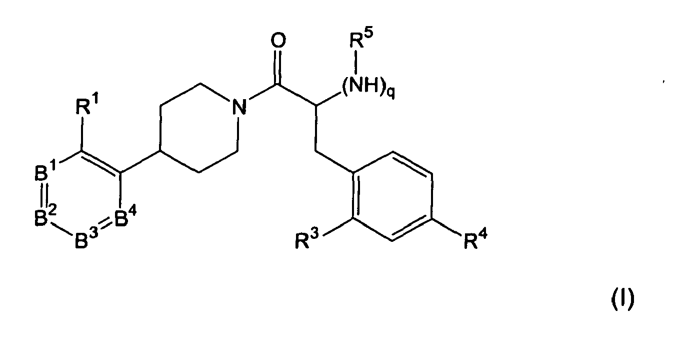 wherein R¹, R³, R⁴, R⁵, B¹, B², B³, B⁴ and q are defined as described below.

The aryl or heteroarylpiperidine derivatives of structural formula (I) are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to substituted aryl or heteroarylpiperidine derivatives useful as melanocortin receptor modulators, in particular, selective MC-4R agonists and MC-4R antagonists.

The compounds of the present invention are represented by structural formula (I) 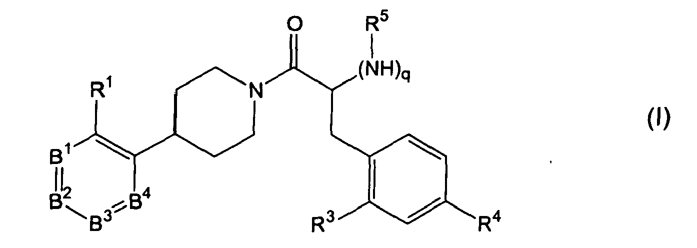 and the enantiomers, diastereomers, tautomers, solvates and pharmaceutically acceptable salts thereof,
wherein
- R¹: is -N(R¹⁰)-(C(R⁶)₂)ₘ-T
-(C(R⁶)₂)_{I}-T, or -O-(C(R⁶)₂)ₘ-T;
- R⁶: is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
- T is: NR⁷R⁸,
morpholine, 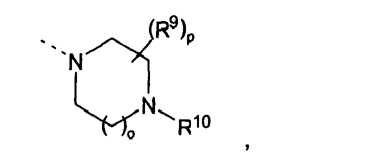 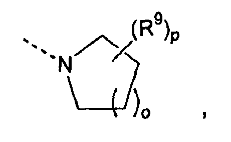 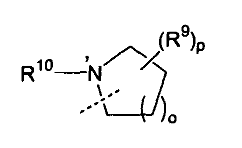 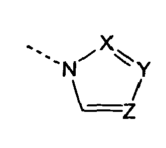 or 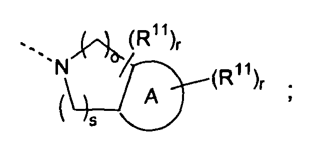
- R⁷ and R⁸: are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
- R⁹: is independently selected from
halogen,
CN,
OH,
C₁₋₆-alkyl optionally substituted with 1 to 3 substituents selected from halogen,
CN and OH,
O-C₁₋₆-alkyl optionally substituted with 1 to 3 substituents selected from halogen, CN and OH, and
C₁₋₆-alkylene-O-C₁₋₆-alkyl optionally substituted with 1 to 3 substituents selected from halogen, CN and OH;
- R¹⁰ is H, or: C₁-C₆-alkyl;
- R¹¹: is independently selected from
halogen,
CN,
OH,
C₁₋₆-alkyl optionally substituted with 1 to 3 substituents selected from halogen,
CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
- X is: CH or N;
- Y: is CH or N;
- Z: is CH or N;
- A: is a 3-7-membered saturated, unsaturated or aromatic ring containing 0-2 nitrogen atoms;
- B¹ to B⁴: are independently selected from CR² and N;
- R²: is independently selected from
H,
F,
Cl,
CH₃,
OCH₃, and
CF₃;
- R³: is H,
Cl,
F, or
CH₃;
- R⁴: is Cl or F;
- R⁵: is 4 to 7-membered heterocyclyl containing 1 or 2 heteroatoms independently selected from the group consisting of NR¹², O and S; or
4 to 7-membered heteroaryl containing 1 to 4 heteroatoms independently selected from the group consisting of N, O and S;
wherein each heterocyclyl may be unsubstituted or substituted by 1 or 2 substituents selected from the group consisting of oxo group, CN, C₁₋₆-alkyl, C₃₋₆-cycloalkyl, halogen and OH, and
each heteroaryl may be unsubstituted or substituted by 1 or 2 substituents selected from the group consisting of C₁₋₆-alkyl, C₃₋₆-cycloalkyl, O-C₁₋₆-alkyl, halogen and CN;
- R¹²: is hydrogen,
C₁₋₆-alkyl,
C₃₋₆-cycloalkyl and
C(O)C₁₋₆-alkyl;
- I: is 0, 1, 2, 3, or 4;
- m: is 0, 1, 2, 3, or 4;
- o: is 0, 1, or 2;
- p: is 0, 1, 2, 3, or 4;
- q: is 0, or 1;
- r: is 0, 1, 2, 3, or 4 and
- s: is 1, or 2.

Preferably, the compounds according to formula (I) adopt the structural conformation of the following stereoisomer formula (I'): 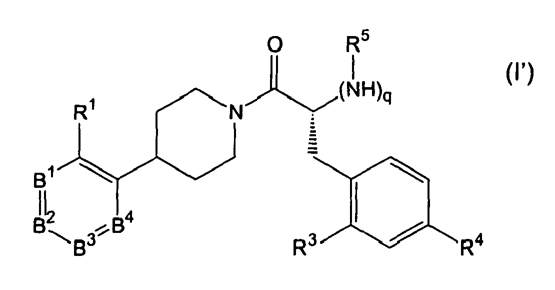 wherein R¹, R³, R⁴, R⁵, B¹, B², B³, B⁴ and q are as defined above.

The moiety 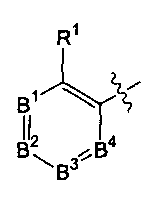 in general formula (I) is selected from the following structures:

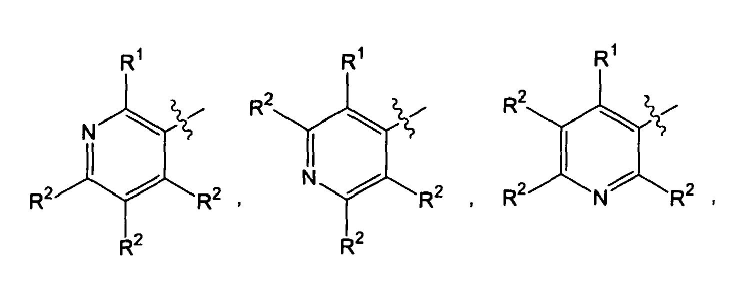

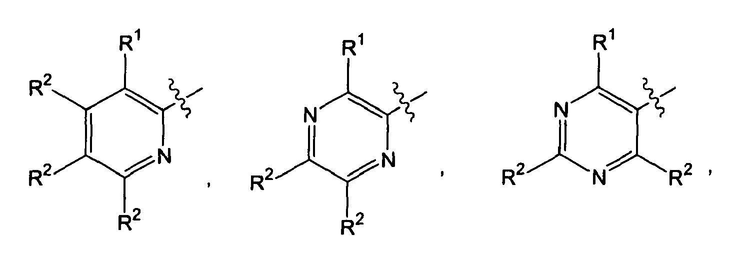

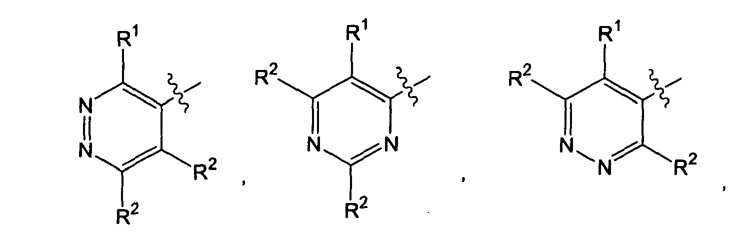

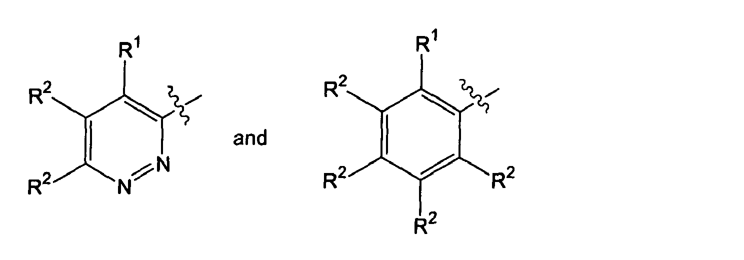

Therein, the variant R² is defined as above. In a preferred embodiment of the present invention, R² represents H, Cl, F or CH₃. More preferred, R² represents H, Cl or F.
It is further preferred, that the variant R² is on the 4-position.

Preferred embodiments of the moiety 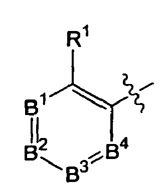 are the following structures:

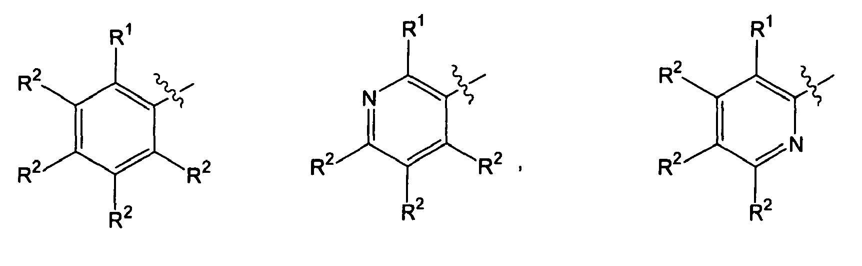

In a preferred embodiment of the present invention, the variant R¹ represents -N(R¹⁰)-(C(R⁶)₂)ₘ-T, -(C(R⁶)₂)_{I}-T, or -O-(C(R⁸)₂)ₘ-T wherein R⁶ and R¹⁰ are preferably independently selected from the group consisting of H and C₁₋₆-alkyl.

It is further preferred that R³ represents H, Cl, or CH₃, more preferably Cl. In an alternative embodiment, R³ preferably represents F.

Preferably, R⁴ represents Cl.

The variant R⁵ is as defined above.
In a preferred embodiment of the present invention, the variant R⁵ represents 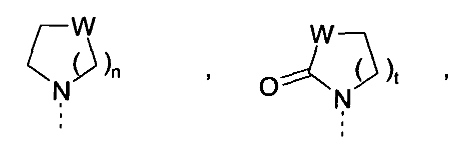 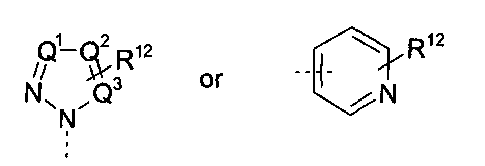 wherein
- W: is CH₂, NR¹², O;
- Q¹, Q², Q³: are independently from each other a nitrogen atom or a carbon atom, with the proviso that at least one of Q¹, Q² and Q³ is a carbon atom;
- R¹²: is hydrogen,
C₁₋₆-alkyl, or
C(O)C₁₋₆-alkyl;
- n: is 0, 1, or 2 and
- t: is 1 or 2.

More preferably, R⁵ is 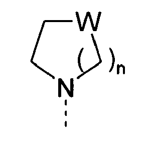 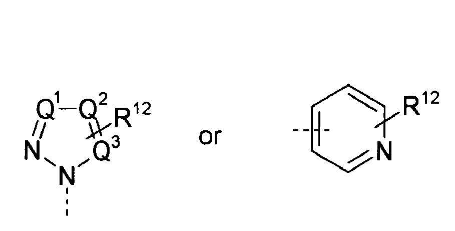 wherein
- n: is 0 to 2;
- W: is CH₂ in the case of n = 0 or 1 and
- W: is CH₂, NR¹² or O in the case of n = 2;
- Q¹, Q², Q³: are independently from each other a nitrogen atom or a carbon atom, with the proviso that at least one of Q¹, Q² and Q³ is a carbon atom; and
R¹² is hydrogen,
C₁₋₆-alkyl, or
C(O)C₁₋₆-alkyl.

Most preferably, R⁵ is selected from the group consisting of the following structures:

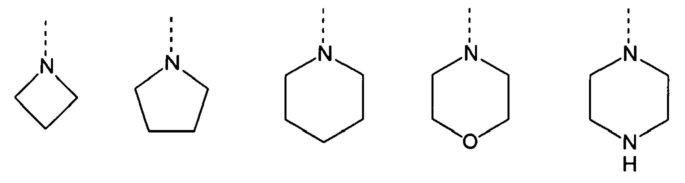

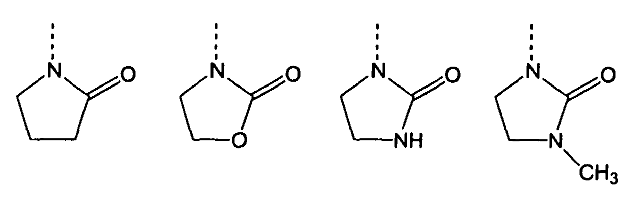

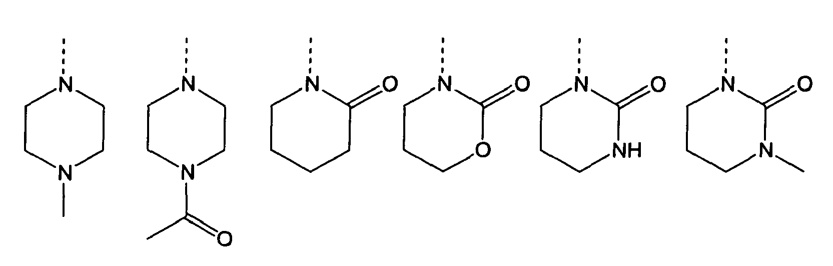

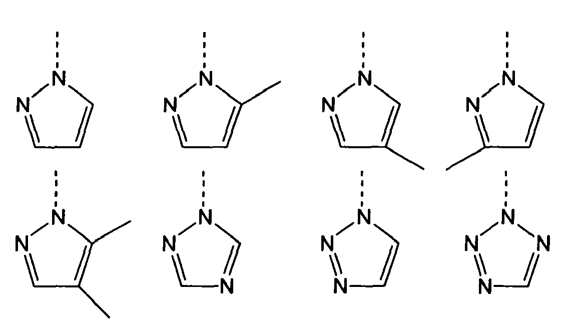

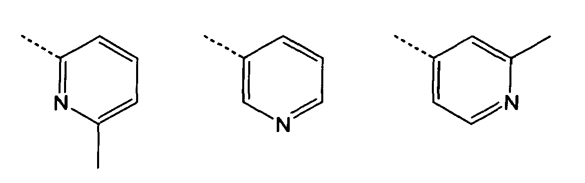

The index q is as defined above. In a preferred embodiment, q assumes the number 0. If R⁵ represents a substituted pyridine group, q preferably assumes the number 1.

In a further preferred embodiment at least one of R⁷ and R⁸ is selected from the group consisting of C₁₋₆-alkyl, C₂₋₆-alkenyl, C₂₋₆-alkinyl and C₂₋₆-alkylene-O-C₁₋₆-alkyl, more preferably from C₂₋₆-alkenyl, C₂₋₆-alkinyl and C₂₋₆-alkylene-O-C₁₋₆-alkyl.

It is preferred that R⁹ is independently selected from the group consisting of halogen, CN, OH, C₁₋₆-alkyl optionally substituted with 1 to 3 substituents selected from halogen, CN and OH, and O-C₁₋₆-alkyl optionally substituted with 1 to 3 substituents selected from halogen, CN and OH.

The variant I is preferably selected from 2 or 3.

The variant m is preferably selected from 2, 3 or 4, more preferably from 2 or 3.

As regards compounds of formula (I), T is preferably selected from the group consisting of the following radicals:

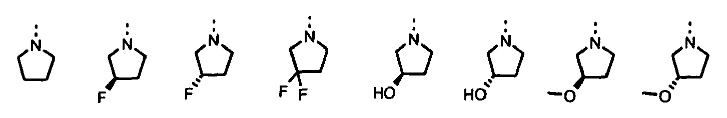

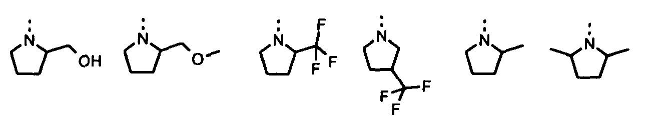

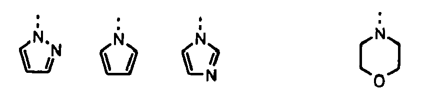

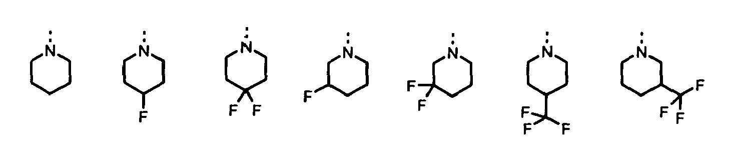

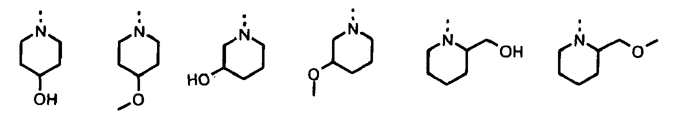

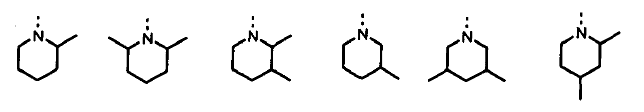

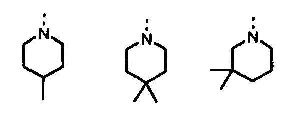

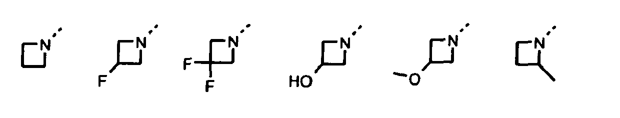

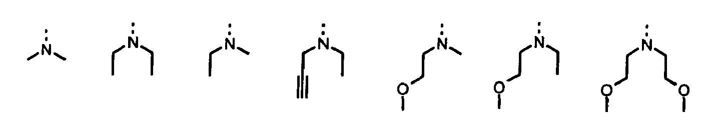

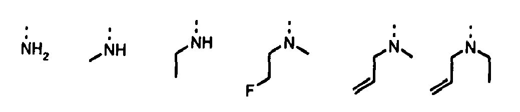

Compounds of the formula (I) in which some or all of the above-mentioned groups have the preferred or more preferred meanings are also an object of the present invention.

In the above and the following, the employed terms have the meaning as described below:
"Alkyl" is a straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl.
"Alkenyl" is a straight chain or branched alkyl having 2 to 6 carbon atoms and which contains at least one carbon-carbon double bond, such as vinyl, allyl, 1-propenyl, 2-butenyl, 2-methyl-2-butenyl, isopropenyl, pentenyl, or hexenyl.
"Alkinyl" is a straight chain or branched alkyl having 2 to 6 carbon atoms and which contains at least one carbon-carbon triple bond, such as ethinyl, 1-propinyl, 1-butinyl, 2-butinyl, pentinyl or hexinyl.

A 3-7-membered, saturated, unsaturated or aromatic ring containing 0-2 nitrogen atoms encompasses a 3-7-membered saturated carbocycle such as cyclopropyl, cyclobutyl, cyclopentyl, cyclohexyl or cycloheptyl. Said term further encompasses 3-7-membered unsaturated carbocycles such as cyclobutenyl, cyclopentenyl, cyclohexenyl, cycloheptenyl, cyclohexa-1,4-diene or cycloheptadienes, or aromatic rings such as benzene. Nitrogen-containing, 3-7-membered, saturated or unsaturated heterocycles and 5-7-membered heteroaryls are further encompassed by the above term. Examples thereof include azetidine, pyrrolidine, piperidine, azepane, piperazine, pyridine, pyrimidine, pyrazine, pyrrole, imidazole, and pyrazole.

The term "heterocyclyl" encompasses a saturated or partially unsaturated, 4 to 7-membered ring containing 1 or 2 heteroatoms selected from the group consisting of N, O and S. In the present invention, a heterocyclyl may be optionally substituted. Examples thereof include azetidine, oxetane, thietane, pyrrolidine, tetrahydrofuran, tetrahydrothiophene, oxazolidine, oxathiolane, thiazolidine, dihydrothiazolidine, piperidine, hexahydropyridazine, hexahydropyrimidine, piperazine, morpholine, pyran, tetrahydropyran, thiopyran, tetrahydrothiopyran, azepane, oxepane, thiepane, or tetrahydro-pyridine. Azetidine, pyrrolidine, tetrahydrofuran, thiazolidine, piperidine, piperazine and morpholine are preferred. Each of said exemplified groups may be optionally substituted.

The term "heteroaryl" encompasses a 4 to 7-membered aromatic ring containing 1 to 4 heteroatoms selected from the group consisting of N, O and S. In the present invention, a heteroaryl may be optionally substituted. Examples thereof include pyrrole, 1*H*-pyrrazole, imidazole, 1*H*-[1,2,3]-triazole, 1*H*-[1,2,4]-triazole, 1*H*-tetrazole, furan, thiophen, thiazole, pyridine, pyrimidine, pyrazine or pyridazine. Pyrrole, 1*H*-pyrrazole, imidazole, 1*H*-[1,2,3]-triazole, 1*H*-[1,2,4]-triazole, 1*H*-tetrazole, pyridine and pyrimidine are preferred. Each of said exemplified groups may be optionally substituted.

The compounds of structural formula (I) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

The compounds of structural formula (I) are particularly useful as antagonists of MC-4R. Thus, they are preferably used for the preparation of a medicament for the treatment and/or prevention of cancer cachexia, muscle wasting, anorexia, anxiety and depression.

### Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic, trifluoroacetic acid and the like.

Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

The compounds of formula (I) are melanocortin receptor modulators and as such are useful in the treatment, control or prevention of diseases, disorders or conditions responsive to the inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance) and male and female sexual dysfunction (including impotence, loss of libido and erectile dysfunction).

The compounds of formulas (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formula (I) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.
Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formula (I), the terms "A moiety", "B moiety" and "C moiety" are used below. This moiety concept is illustrated below:

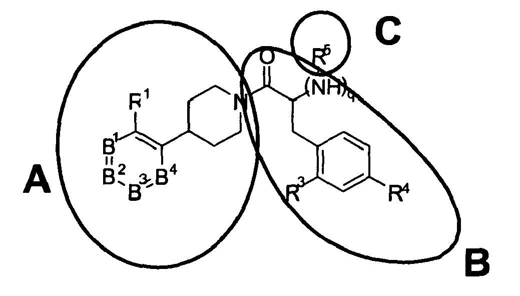

The preparation of the compounds of the present invention may be carried out via sequential or convergent synthetic routes. The skilled artisan will recognize that, in general, the A and B moieties of a compound of formula (I) are connected via amide bonds. The skilled artist can, therefore, readily envision numerous routes and methods of connecting the two moieties via standard peptide coupling reaction conditions.

The phrase "standard peptide coupling reaction conditions" means coupling a carboxylic acid with an amine using an acid activating agent such as EDCI, dicyclohexylcarbodiimide and benzotriazol-1-yloxytris(dimethylamino)-phosphonium hexafluorophosphate, in a inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following schemes and examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation.
The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings:
- AcOH: acetic acid
- Ac₂O: acetic anhydride
- Boc: tert-butoxycarbonyl
- Boc₂O: di-tert-butyl dicarbonate
- BuLi: butyllithium
- Bz₂O₂: dibenzoylperoxide
- DCM: dichloromethane
- DEAD: diethyl azodicarboxylate
- DIBAL-H: diisobutylaluminumhydride
- DIAD: diisopropyl azodicarboxylate
- DIEA: ethyl-diisopropylamine
- DMA: N,N-dimethylacetamide
- DMAP: 4-dimethylaminopyridine
- DMF: N,N-dimethylformamide
- DMS: dimethylsulfide
- dppf: 1,1'-bis(diphenylphosphino)-ferrocen
- EDCI: 1-(3-dimethylaminopropyl)-3-ethylcarbodiimide hydrochloride
- Et₂O: diethyl ether
- EtOAc: ethyl acetate
- EtOH: ethanol
- h: hour(s)
- HATU: O-(7-azabenzotriazol-1-yl)-N,N,N',N'-tetramethyluronium hexafluorophosphate
- HOAt: 1-hydoxy-7-azabenzotriazole
- HOBt: 1-hydroxybenzotriazole hydrate
- HTMP: 2,2,6,6-tetramethylpiperidine
- MeCN: acetonitrile
- MeOH: methanol
- min: minute(s)
- NBS: N-bromosuccinimide
- NMM: N-methylmorpholine
- PG: protecting group
- PPh₃: triphenylphosphine
- RT: room temperature
- TEA: triethylamine
- Tf: trifluoromethanesulfonyl
- (Tf)₂O: trifluoromethanesulfonic anhydride
- TFA: trifluoroacetic acid
- THF: tetrahydrofurane
- TMSCHN₂: trimethylsilyldiazomethane

### Reaction scheme 1:

### Synthesis of Phenylpiperidine Derived A Moieties with Alkylether Spacer (R¹ = -O(C(R⁶)₂)ₘ-T)

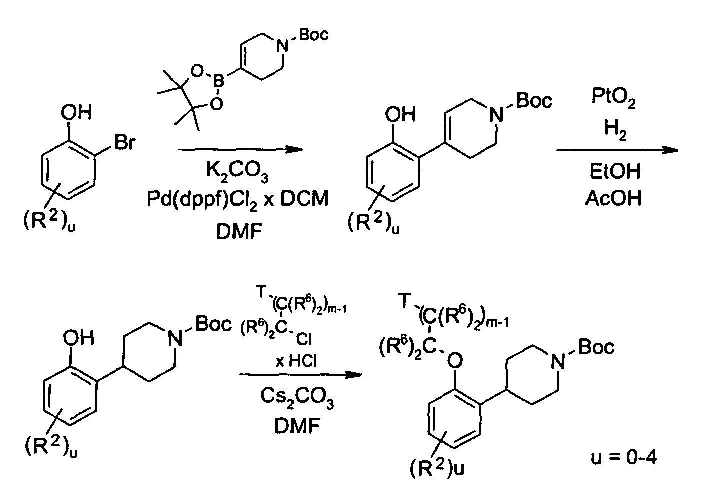

As shown in Reaction Scheme 1, optionally substituted 2-bromo-phenol and 4-(4,4,5,5-tetramethyl-[1,3,2]dioxaborolan-2-yl)-3,6-dihydro-2H-pyridine-1-carboxylic acid tert-butyl ester (Tetrahedron Lett. 2000, 41, 3705-3708) are reacted in a Suzuki coupling in the presence of a base such as K₂CO₃ and a catalyst such as dichloro(1,1'-bis(diphenyl-phosphino)-ferrocene)palladium(II) DCM adduct, in an organic solvent such as DMF or toluene, at a suitable temperature. The resulting tetrahydropyridine can be hydrogenated in the presence of a catalyst, such as PtO₂ or Pd/C, to yield the protected piperidine. The piperidine can be further reacted with an alkylchloride or alkylbromide bearing the capping group T in the presence of a base such as Cs₂CO₃ or NaH in an appropriate solvent such as DMF to give the Boc-protected A moiety.

### Reaction scheme 2:

### Alternative Synthesis of Phenylpiperidine Derived A Moieties with Alkylether Spacer (R¹ = -O(C(R⁶)₂)ₘ-T)

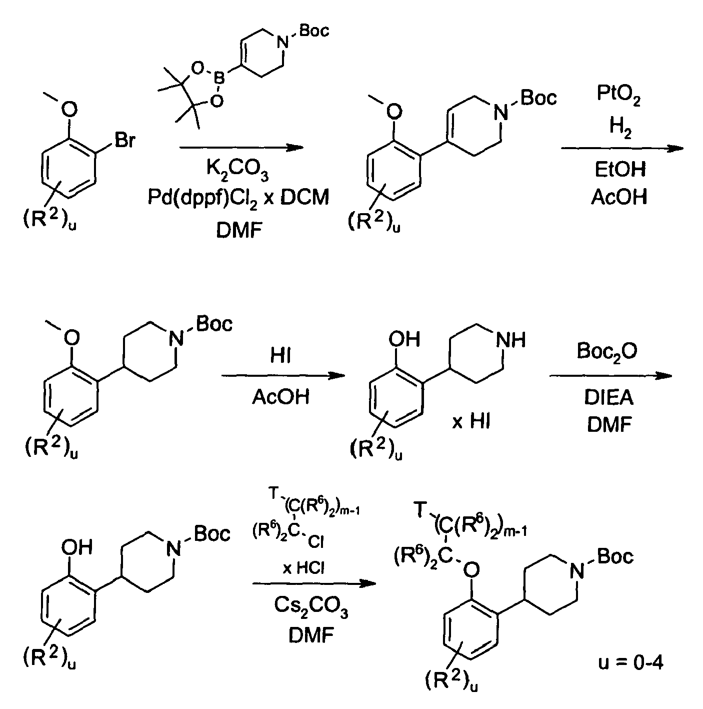

The synthesis of phenylpiperdine derived A Moieties bearing an alkylether spacer (R¹ = -O(C(R⁶)₂)ₘ-T) can alternatively be performed starting from optionally substituted 2-bromoanisole (see Reaction scheme 2). A Suzuki coupling with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature leads to the corresponding tetrahydropyridine. The resulting tetrahydropyridine can be hydrogenated in the presence of a catalyst, such as PtO₂ or Pd/C, to yield the protected piperidine. The methylether can be cleaved with a reagent such as aqueous hydroiodic acid in acetic acid or trimethylsilyl iodide in chloroform, at a suitable temperature to get access to the corresponding phenol as hydroiodide. The Boc-protecting group, which is lost during this process, can subsequently be reintroduced by using a reagent such as Boc₂O in the presence of a base such as DIEA in an appropriate solvent such as DCM or DMF. The Boc-protected piperidine can be further reacted with an alkylchloride or alkylbromide bearing the capping group T in the presence of a base such as Cs₂CO₃ or NaH in an appropriate solvent such as DMF to give the Boc-protected A moiety.

### Reaction scheme 3: Synthesis of Phenylpiperidine Derived A Moieties with Alkylether Spacer (R¹ =

### -O(C(R⁶)₂)ₘ-T) Using Mitsunobu Conditions

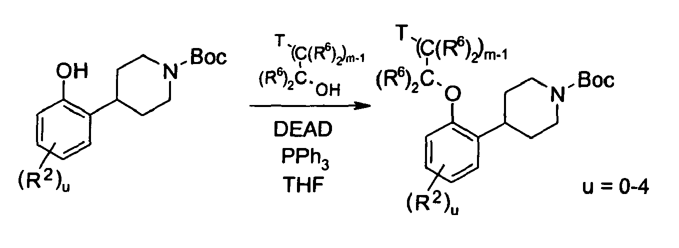

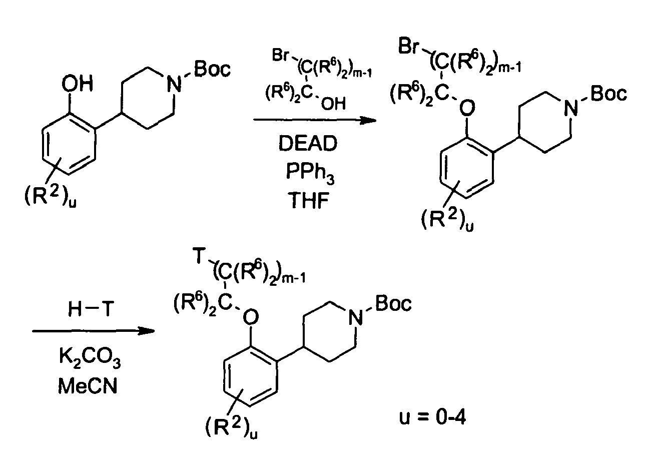

As shown in Reaction scheme 3, the intermediate product from Reaction schemes 1 and 2, optionally substituted 1-Boc-4-(2-hydroxy-phenyl)-piperidine, can also be alkylated with an ω-T-capped alkylalcohol in the presence of a reagent such as DEAD or DIAD and a phosphine such as PPh₃ in a suitable solvent such as THF to give the Boc-protected A moieties.

Similarly, the same intermediate can be reacted with an ω-bromo alkylalcohol, using the reaction conditions described above, to give access to the corresponding phenolether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

### Reaction scheme 4:

### Synthesis of Phenylpiperidine Derived A Moieties with Alkylene Spacer (R¹ = -(C(R⁶)₂)_{I}-T,I=3)

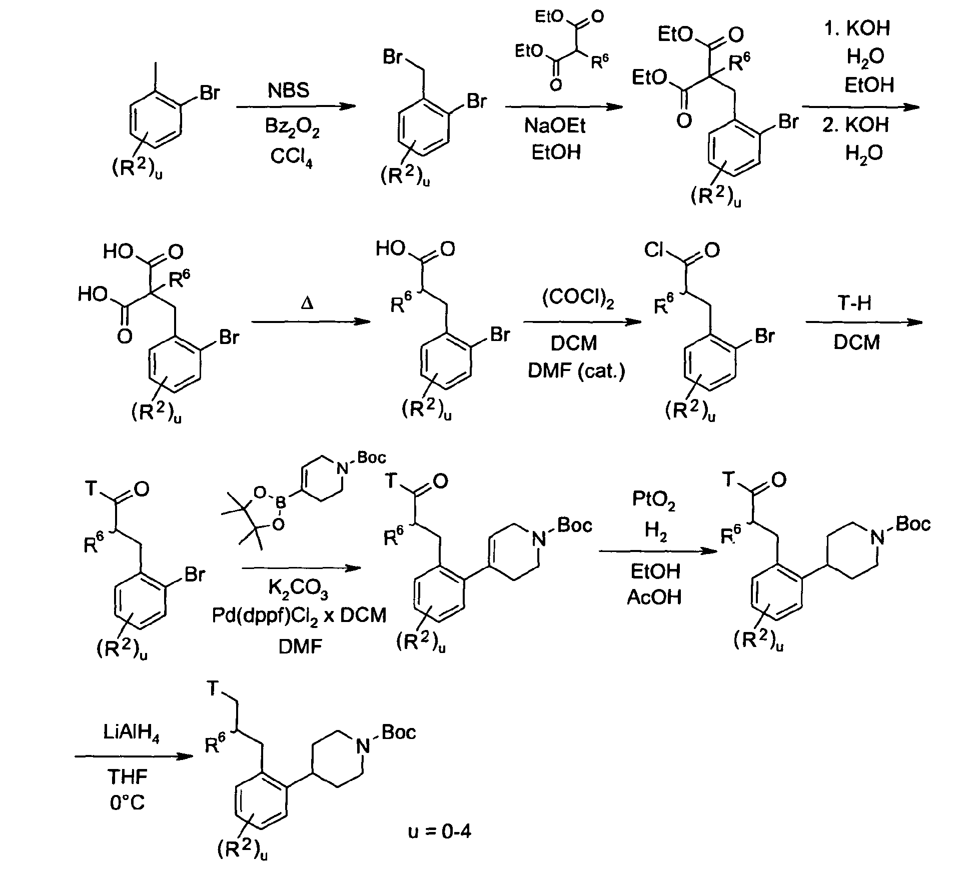

The first route for the synthesis of phenylpiperidine derived A moieties bearing an alkylene spacer (R¹ = -(C(R⁶)₂)_{I}-T) is depicted in Reaction scheme 4. Optionally substituted 2-bromotoluene is brominated with NBS in the presence of a radical starter such as Bz₂O₂ in an appropriate solvent such as CCl₄ at a suitable temperature to yield the corresponding benzylbromide. The benzylbromide is reacted with optionally substituted diethyl malonate in the presence of a base such as sodium ethoxide in a suitable solvent such as ethanol. Subsequent saponification with a base such as KOH in an appropriate solvent such as water-ethanol mixture followed by a second saponification step with a suitable base such as KOH in a solvent such as water leads to the alkylated malonic acid which is decarboxylated at an appropriate temperature. The product of this reaction, optionally substituted 3-(2-bromophenyl)propionic acid, is converted to the acid chloride using a reagent such as oxalyl chloride or thionyl chloride in an inert solvent such as DCM with a catalytic amount of DMF, and reacted with the capping group T to form the corresponding amide. Optionally substituted 3-(2-bromophenyl)propionic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

### Reaction scheme 5:

### Alternative Route for the Synthesis of Phenylpiperdine Derived A Moieties with Alkylene Spacer (R¹ = -(CH₂)_{I}-T, I = 3)

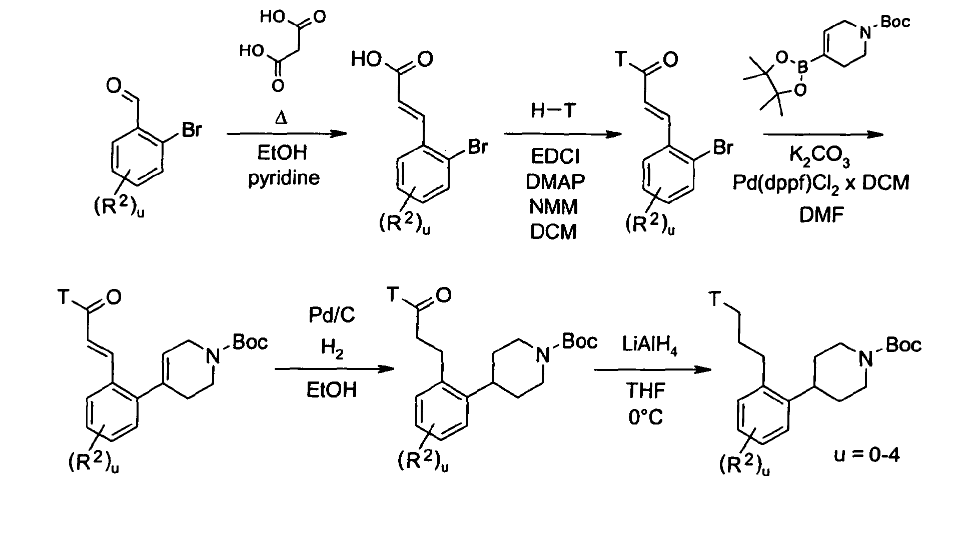

An alternative approach for the synthesis of of phenylpiperidine derived A moieties bearing an alkylene spacer (R¹ = -(CH₂)_{I}-T) starts with optionally substituted 2-bromobenzaldehyde (see Reaction scheme 5). Reaction with malonic acid in an appropriate solvent such as ethanol, in the presence of a base such as pyridine, at a suitable temperature, leads to the corresponding 2'-bromo-cinnamic acid. Said acid is activated with a reagent such as EDCI in the presence of a catalyst such as DMAP and a base such as NMM in DCM, and reacted with the capping group T to form the corresponding amide. Optionally substituted 2'-bromo-cinnamic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine and the cinnamic acid amide double bond can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

### Reaction scheme 6:

### Alternative Route for the Synthesis of Phenylpiperidine Derived A Moieties with Alkylene Spacer (R¹ = -(C(R⁶)₂)_{I}-T, I = 3)

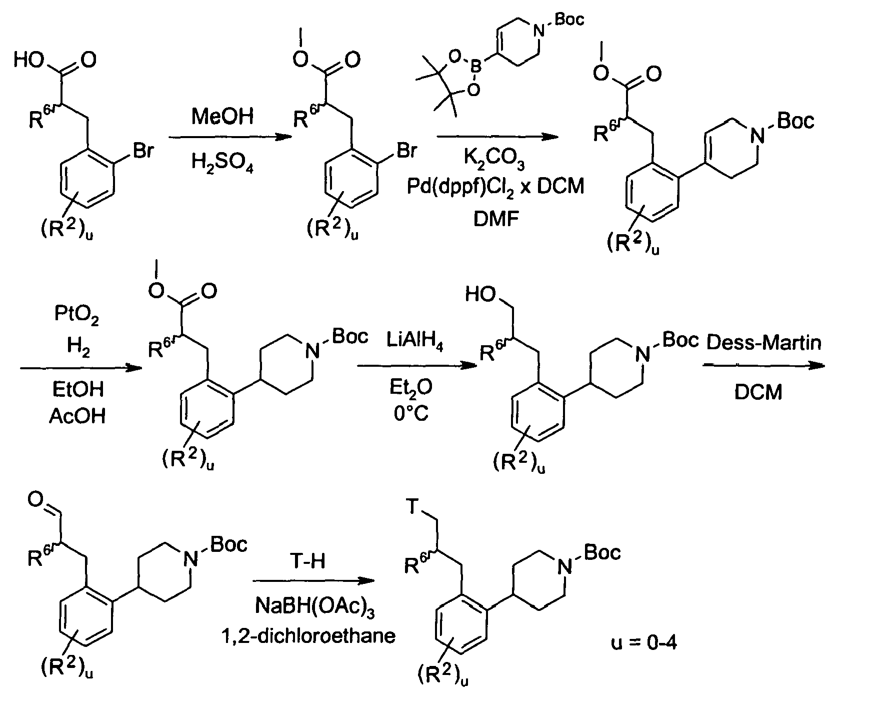

As shown in Reaction scheme 6, optionally substituted 3-(2-bromophenyl)propionic acid, is reacted with methanol in the presence of a catalyst such as sulfuric acid to form the corresponding methyl ester. Optionally substituted 3-(2-bromophenyl)propionic acid ester can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichioro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain ester function can be reduced using a reagent such as LiAlH₄ or borane-THF complex in an appropriate inert solvent such as diethyl ether or THF at a suitable temperature to yield the corresponding alcohol which can subsequently be oxidized using a reagent such as Dess-Martin periodinane in an appropriate solvent such as DCM or using sulfurtrioxide-pyridine complex with a base such as triethylamine in a suitable solvent such as DCM. Optionally substituted 3-(2-bromophenyl)propionyl aldehyde is reacted with the capping group T in the presence of a reducing agent such as sodium triacetoxyborohydride in an appropriate solvent such as 1,2-dichloroethane to form the corresponding Boc-protected A moiety.

### Reaction scheme 7:

### Synthesis of Phenylpiperidine Derived A Moieties with Alkylene Spacer (R¹ = -(C(R⁶)₂)_{I}-T, I = 3) Using a Negishi Coupling Reaction

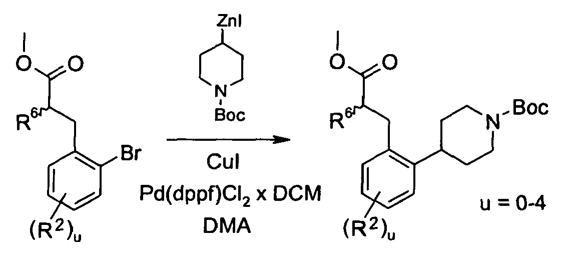

As shown in Reaction scheme 7, the intermediate product from Reaction scheme 6, optionally substituted 3-(2-bromophenyl)propionic acid ester can also be subjected to a Negishi coupling with (1-tert-butoxycarbonylpiperidin-4-yl)(iodo)zinc (J. Org. Chem. 2004, 69, 5120-5123) in the presence of copper(I) iodide and dichloro(1,1'-bis(diphenyl-phosphino)-ferrocene)palladium(II) DCM adduct in an inert solvent such as DMA to yield the resulting phenylpiperidine which can be further processed as shown in Reaction scheme 6.

### Reaction scheme 8:

### Alternative Route for the Synthesis of Phenylpiperidine Derived A Moieties with Alkylene Spacer (R¹ = -(C(R⁶)₂)_{I}-T, I = 2, 3)

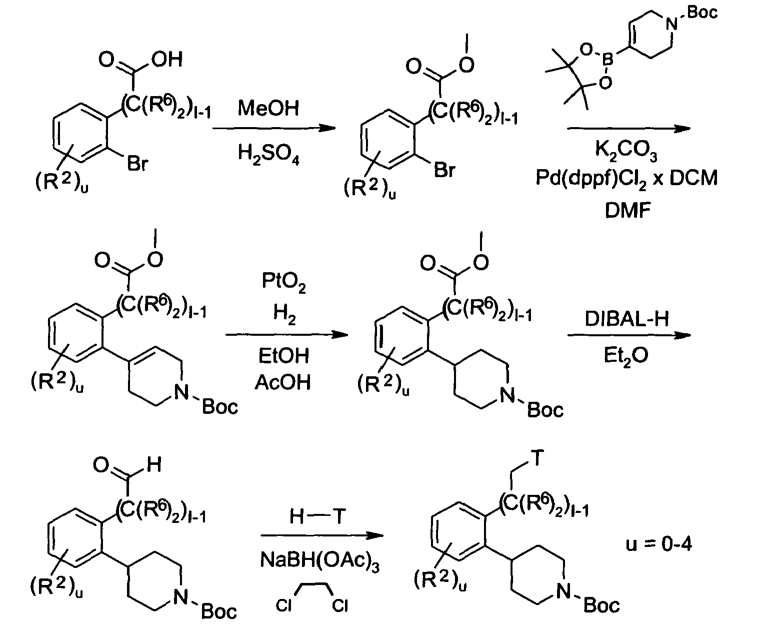

As shown in Reaction scheme 8 optionally substituted 3-(2-bromophenyl)propionic acid or 2-(2-bromophenyl)acetic acid is transformed to the corresponding methyl ester using a catalyst such as sulfuric acid. The ester can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine.
The resulting tetrahydropyridine can be hydrogenated in the presence of a catalyst, such as PtO₂ or Pd/C, to yield the protected piperidine. The ester function can then be reduced to the corresponding aldehyde with DIBAL-H in an appropriate solvent such as Et₂O or THF at a suitable temperature. Reductive amination of the aldehyde with an amine T-H in the presence of a reducing agent such as sodium triacetoxyborohydride in an appropriate solvent such as 1,2-dichloroethane leads to the Boc-protected A moiety.

### Reaction scheme 9:

### Synthesis of Phenylpiperidine Derived A Moieties with Alkylene Spacer (R¹ = -(C(R⁶)₂)_{I}-T, I = 2)

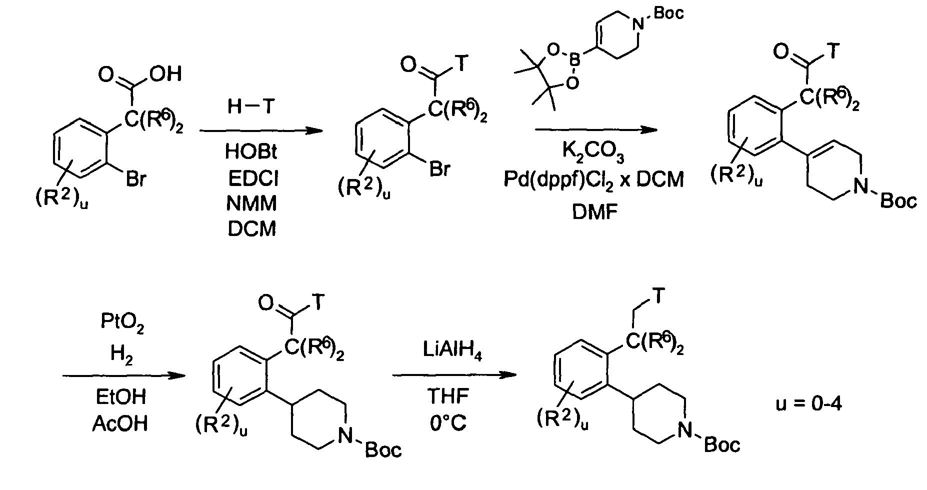

Synthesis of phenylpiperidine derived A Moieties with alkylene spacer (R¹ = -(C(R⁶)₂)_{I}-T, I = 2) can also be performed as described in Reaction scheme 9. Optionally substituted 2'-bromophenylacetic acid can be activated with a reagent such as EDCI in the presence of a catalyst such as DMAP and a base such as NMM in DCM, and reacted with the capping group T to form the corresponding amide. Optionally substituted 2'-bromo-phenylacetic amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAIH₄ or borane-THF complex in an appropriate inert solvent such as diethyl ether or THF at a suitable temperature to yield the Boc-protected A moiety.

### Reaction scheme 10:

### Synthesis of Phenylpiperidine Derived A Moieties with Alkylene Spacer (R¹ = -(C(R⁶)₂)_{I}-T, I = 4)

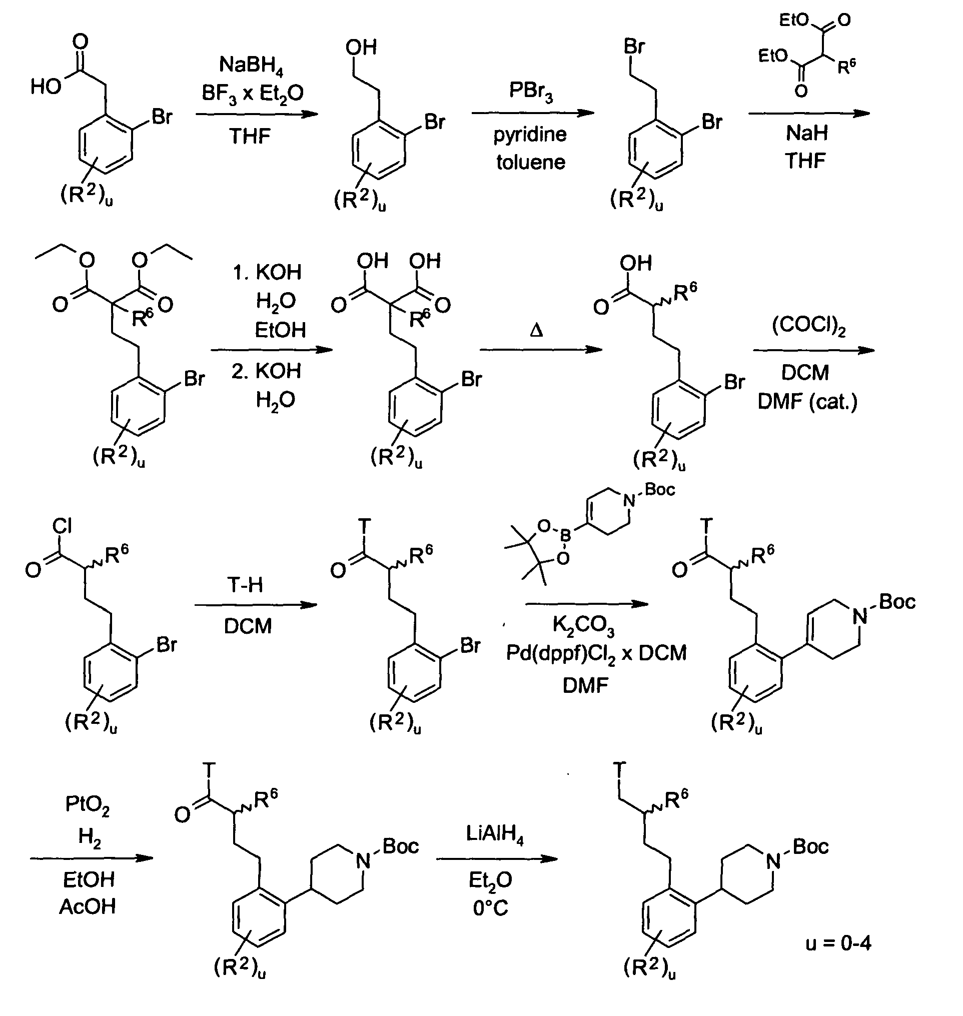

A route for the synthesis of phenylpiperidine derived A moieties bearing an C₄-alkylene spacer (R¹ = -(C(R⁶)₂)_{I}-T, I = 4) is depicted in Reaction scheme 10. Optionally substituted 2-bromophenylacetic acid can be reduced with sodium borohydride in the presence of a reagent such like boron trifluoride diethyl etherate in an appropriate solvent such as THF at a suitable temperature to yield the corresponding phenylethylalcohol. Reaction of the alcohol with a bromination reagent such as phosphorous tribromide in the presence of a base such as pyridine in an appropriate solvent like toluene at a suitable temperature leads to the phenylethylbromide. The phenethylbromide can be reacted with optionally substituted diethyl malonate in the presence of a base such as sodium hydride in a suitable solvent such as THF. Subsequent saponification with a base such as KOH in an appropriate solvent such as water-ethanol mixture followed by a second saponification step with a suitable base such as KOH in a solvent such as water leads to the alkylated malonic acid which can be decarboxylated at an appropriate temperature. The product of this reaction, optionally substituted 3-(2-bromophenyl)butanoic acid, can be converted to the acid chloride using a reagent such as oxalyl chloride or thionyl chloride in an inert solvent such as DCM with a catalytic amount of DMF, and reacted with the capping group T to form the corresponding amide. Optionally substituted 3-(2-bromophenyl)butanoic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

### Reaction scheme 11

### Synthesis of Phenylpiperidine Derived A Moieties Containing Cyclic Amines

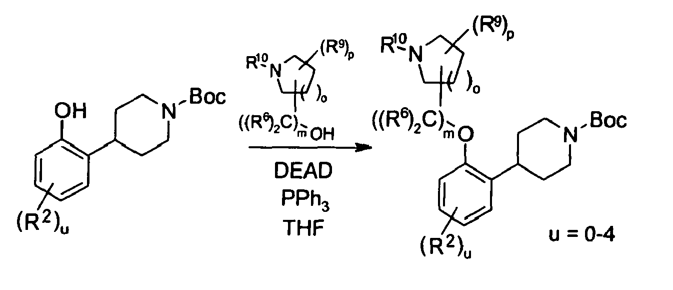

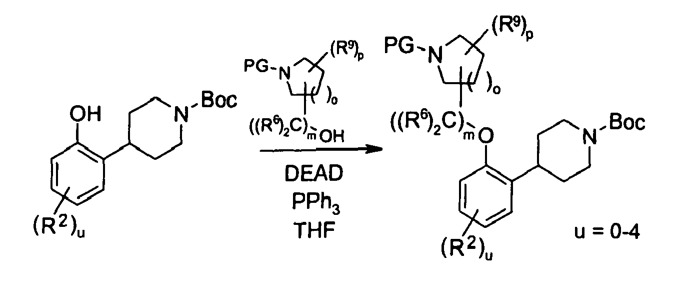

As shown in Reaction scheme 11, the intermediate product from Reaction schemes 1 and 2, optionally substituted 1-Boc-4-(2-hydroxy-phenyl)-piperidine, can also be alkylated with an alcohol which contains a cyclic tertiary amine moiety in the presence of a reagent such as DEAD or DIAD and a phosphine such as PPh₃ in a suitable solvent such as THF to give the Boc-protected A moieties.

Similarly, an alcohol containing a protected cyclic secondary amine moiety can be introduced as building block using the conditions described above. The protecting group has to be orthogonal to the Boc-protecting group used for protection of the piperidine. After coupling of the A moiety with the B-C moiety this protecting group can be removed using standard methods.

### Reaction scheme 12:

### Directed ortho-metallation

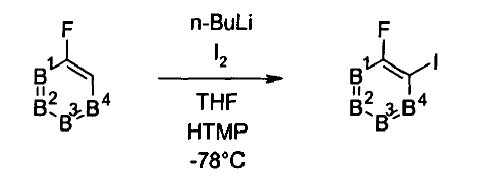

The starting material for the synthesis of heteroarylpiperidines, ortho-fluoro-iodopyridines, -pyridazines and -pyrazines, can be prepared as shown in Reaction scheme 12. A fluoro-substituted heteroaryl can be metallated with an amide prepared from a reagent such as n-butyllithium and an amine such as diisopropylamine or 2,2,6,6-tetramethylpiperidine at an appropriate temperature in a suitable solvent such as THF. The resulting lithio derivatives can subsequently be reacted with iodine to yield the desired compounds.

### Reaction scheme 13:

### Acetylation

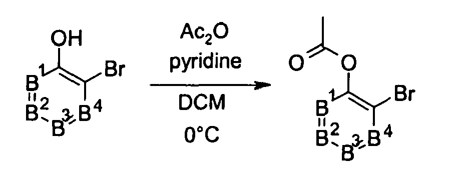

Another starting material for the synthesis of heteroarylpiperidines, ortho-acetoxy-bromopyridines, -pyridazines and -pyrazines, can be prepared as shown in Reaction scheme 13. A bromoheteroaryl containing a hydroxy group in ortho-position to the bromo atom can be reacted with an acetylating reagent such as acetic anhydride in the presence of a suitable base such as pyridine in an appropriate solvent like DCM at a suitable temperature.

### Reaktion scheme 14:

### Negishi Coupling Reaction of Bromo- or Iodosubstituted Heteroaryls

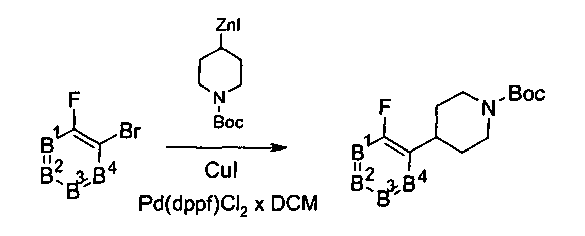

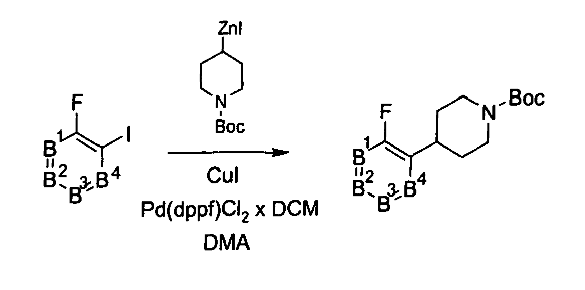

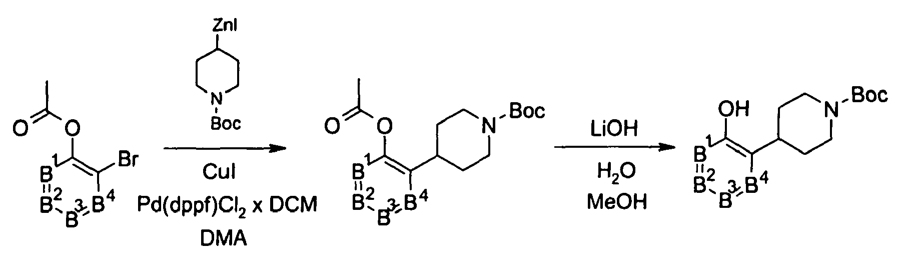

As shown in Reaction scheme 14, ortho-fluoro-bromopyridines, pyridazines and -pyrazines can be subjected to a Negishi coupling with (1-tert-butoxycarbonylpiperidin-4-yl)(iodo)zinc (J. Org. Chem. 2004, 69, 5120-5123) in the presence of copper(I) iodide and dichloro(1,1'-bis(diphenyl-phosphino)-ferrocene)palladium(II) DCM adduct in an inert solvent such as DMA to yield the resulting heteroarylpiperidine. The same product is obtained, when ortho-fluoro-iodopyridines, -pyridazines and -pyrazines are used as starting material.
The Negishi coupling can alternatively be performed using the ortho-acetoxysubstituted bromopyridines, -pyridazines and -pyrazines from Reaction scheme 2 as starting material. The free alcohol can be obtained by saponification of the acetic acid ester with a base such as lithium hydroxide in a suitable solvent such as mixture of water and methanol.

### Reaktion scheme 15:

### Nucleophilic Aromatic Substitution Reaction

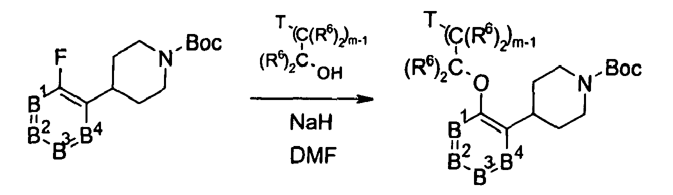

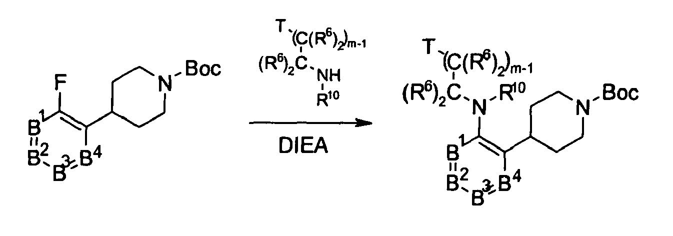

As shown in Reaction scheme 15, fluoro-substituted pyridyl-, pyridazyl- and pyrazinylpiperidines can be subjected to a nucleophilic aromatic substitution reaction with a ω-T-capped alkylalcohol in the presence of a base such as sodium hydride in a solvent such as DMF at a suitable temperature to obtain the Boc-protected A moiety.
Alternatively, the fluoro-substituted heteroarylpiperidines can also be reacted with a ω-T-capped primary or secondary alkylamine in the presence of a base like BuLi or DIEA in an appropriate solvent like THF or without a solvent at a suitable temperature to obtain the Boc-protected A moiety.

### Reaction scheme 16

### Synthesis of Heteroarylpiperidine Derived A Moieties Containing Cyclic Amines

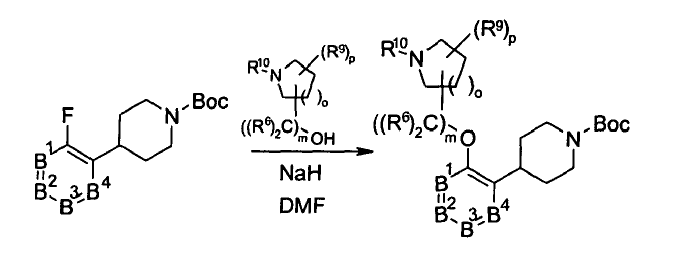

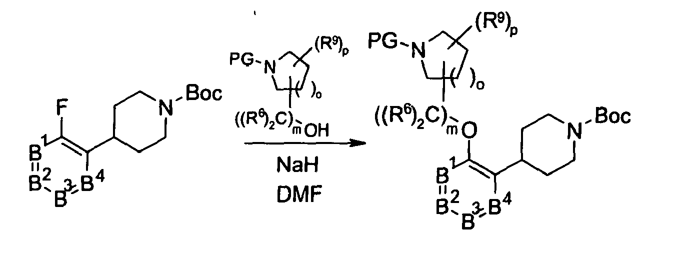

As shown in Reaction scheme 16, the intermediate product from Reaction scheme 14, optionally substituted fluoropyridines, -pyridazines and -pyrazines, can also be subjected to a nucleophilic aromatic substitution reaction with an alcohol which contains a cyclic tertiary amine moiety, in the presence of a base such as sodium hydride in a suitable solvent such as DMF to give the Boc-protected A moieties.

Similarly, an alcohol containing a protected cyclic secondary amine moiety can be introduced as building block using the conditions described above. The protecting group has to be orthogonal to the Boc-protecting group used for protection of the piperidine. After coupling of the A moiety with the B-C moiety this protecting group can be removed using standard methods.

### Reaction scheme 17:

### Alternative Synthesis of Heteroarylpiperidine Derived A Moieties with Alkylether Spacer (R¹ = -O(C(R⁶)₂)ₘ-T)

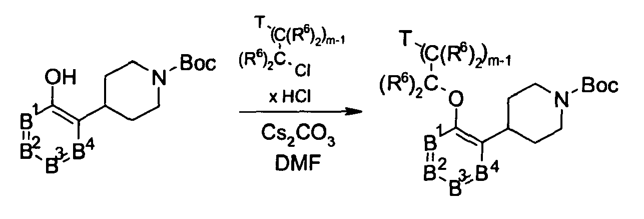

The synthesis of heteroarylpiperidine derived A Moieties bearing an alkylether spacer (R¹ = -O(C(R⁶)₂)ₘ-T) can alternatively be performed as depicted in Reaction scheme 17. The Boc-protected piperidine can be reacted with an alkylchloride or alkylbromide bearing the capping group T in the presence of a base such as Cs₂CO₃ or NaH in an appropriate solvent such as DMF to give the Boc-protected A moiety.

### Reaction scheme 18:

### Synthesis of Heteroarylpiperidine Derived A Moieties with Alkylether Spacer (R¹ = -O(C(R⁶)₂)ₘ-T) Using Mitsunobu Conditions

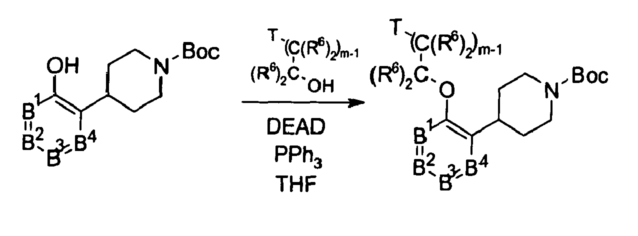

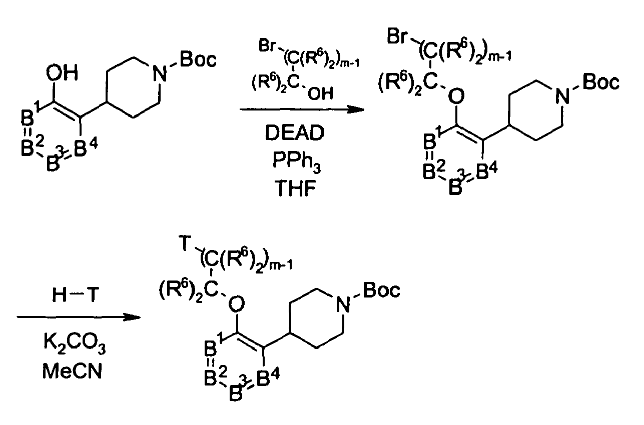

As shown in Reaction scheme 18, the intermediate product from Reaction scheme 14, optionally substituted (hydroxyheteraoaryl)piperidines, can also be alkylated with an ω-T-capped alkylalcohol in the presence of a reagent such as DEAD or DIAD and a phosphine such as PPh₃ in a suitable solvent such as THF to give the Boc-protected A moieties.

Similarly, the same intermediate can be reacted with an ω-bromo alkylalcohol, using the reaction conditions described above, to give access to the corresponding ether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

### Reaction scheme 19

### A Moiety Deprotection

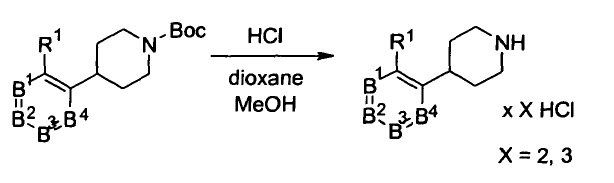

Generally, the starting material of Boc-protected phenylpiperidine or heteroarylpiperidine (A moiety) can be deprotected in the presence of TFA/CH₂Cl₂, HCl/EtOAc, HCl/dioxane or HCl in MeOH/dioxane with or without a cation scavenger, such as dimethyl sulfide (DMS) before being subjected to the coupling procedure. It can be converted to the free base before being subjected to the coupling procedure or in some cases used as the salt.

### Reaction scheme 20:

### Formation of B-C Moieties Containing Heterocyclyls ((NH)_{q}-R⁵, q = 0)

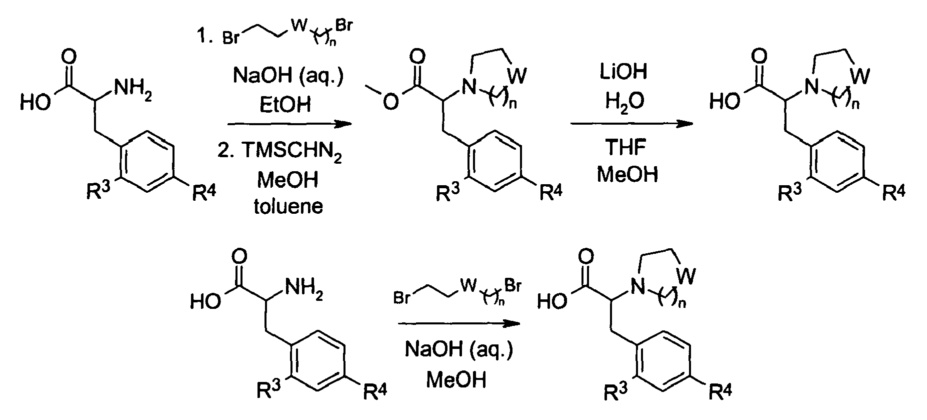

The B-C moieties containing heterocyclyls as R⁵ can be synthesized as shown in Reaction scheme 20. Optionally substituted phenylalanine can be reacted with a dibromoalkane, a dibromoalkylether, -thioether or -amine in a solvent such as ethanol or methanol in the presence of a base such as aqueous sodium hydroxide at an appropriate temperature to form the B-C- moiety. For improved purification this can be converted to the corresponding methyl ester with a reagent such as trimethylsilyldiazomethan in a suitable solvent like toluene in the presence of a proton source like methanol. Following purification the ester function can be hydrolyzed with a base such as LiOH in a suitable solvent or solvent mixture such as water/THF/methanol to give access to the B-C moiety.
In some cases the pure B-C moiety can also be obtained directly by purification of the alkylation product.

### Reaction scheme 21:

### Alternative Method for the Formation of B-C Moieties Containing Heterocyclyls ((NH)_{q}-R⁵, q = 0)

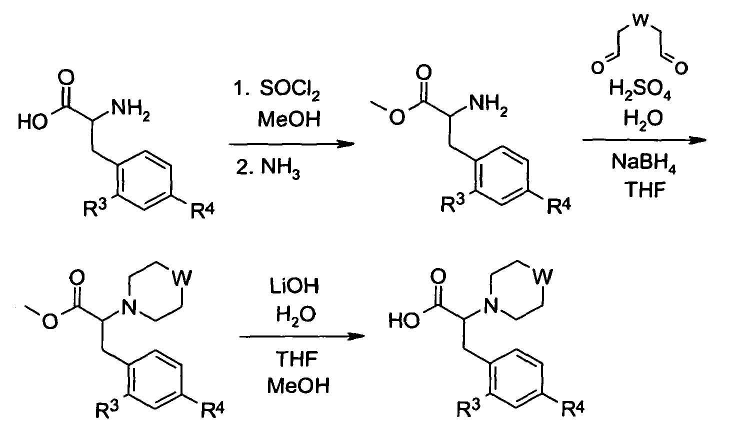

The heterocyclyl containing B-C moieties can alternatively be synthesized as shown in Reaction scheme 21. Optionally substituted phenylalanine can be converted to the corresponding methyl ester hydrochloride using an activating reagent such as thionyl chloride or oxalyl chloride in methanol. The free amino acid methyl ester can be obtained by treatment of the hydrochloride with a base such as aqueous ammonia. Subsequent reductive amination with an aliphatic dialdehyde hydrochloride using a reducing agent such as sodium borohydride in the presence of sulfuric acid in an appropriate solvent such as a mixture of water and THF leads to the B-C moiety methyl ester. The ester function can be hydrolyzed with a base such as LiOH in a suitable solvent or solvent mixture such as water/THF/methanol to give access to the B-C moiety.

### Reaction scheme 22:

### Formation of B-C Moieties Containing Heteroaryls ((NH)_{q}-R⁵, q = 0)

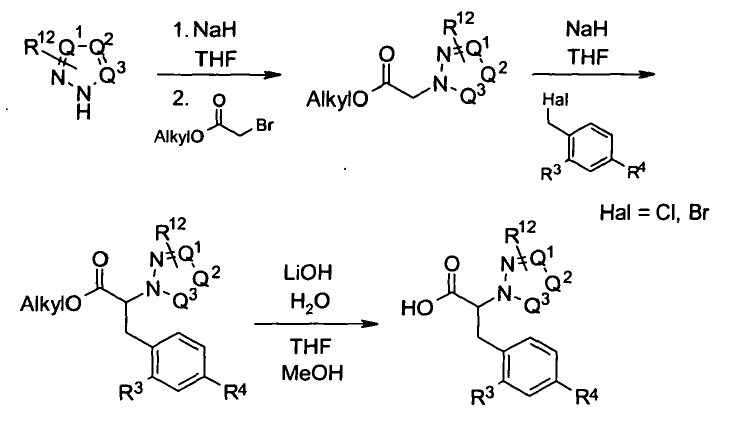

The B-C moieties containing heteroaryls as R⁵ can be synthesized as shown in Reaction scheme 22. Optionally substituted nitrogen containing heterocycle is treated with a base such as sodium hydride in an appropriate solvent such as THF or DMF to yield the corresponding sodium salt which can subsequently be reacted with a bromoacetic acid ester. The product of this reaction, optionally substituted heteroarylacetic acid ester, can be reacted with optionally substituted benzylbromides and -chlorides in the presence of a base such as sodium hydride in a suitable solvent such as THF or DMF at an appropriate temperature. The ester function can be hydrolyzed with a base such as LiOH in a suitable solvent or solvent mixture such as water/THF/methanol to give access to the B-C moiety. In some cases the ester can also be cleaved using acidic conditions.

### Reaction scheme 23:

### Alternative Method for the Formation of B-C Moieties Containing Heteroaryls ((NH)_{q}-R⁵, q = 0)

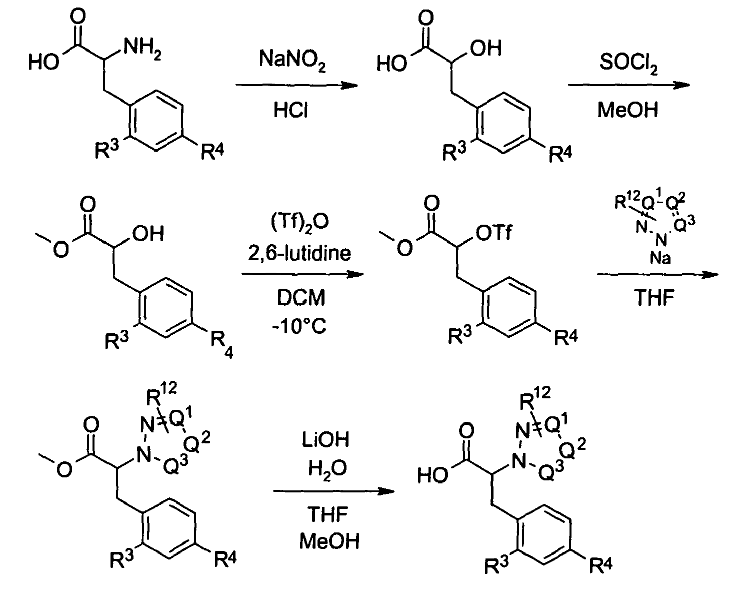

The B-C moieties containing heteroaryls as R⁵ can alternatively be synthesized as shown in Reaction scheme 23. Optionally substituted phenylalanine can be subjected to a diazotization reaction with sodium nitrite in the presence of aqueous hydrochloric acid to yield the corresponding α-hydroxyacid with retention of configuration at the α-carbon (J. Org. Chem. 2001, 66, 7303-7312). The resulting α-hydroxyacid can be converted to the corresponding methyl ester using an activating reagent such as thionyl chloride or oxalyl chloride in methanol which can subsequently be reacted with trifluoromethanesulfonic anhydride in the presence of a base such as 2,6-lutidine in a suitable solvent like DCM at an appropriate temperature. Optionally substituted 3-phenyl-2-trifluoromethanesulfonyloxy-propionic acid methyl ester can be reacted with the sodium salt of the nitrogen containing heterocycle prepared as desribed in Reaction scheme 22, in an appropriate solvent such as THF or DMF at a suitable temperature. The ester function can be hydrolyzed with a base such as LiOH in a suitable solvent or solvent mixture such as water/THF/methanol to give access to the B-C moiety.

### Reaction scheme 24:

### Formation of B-C Moieties Containing Aminopyridines ((NH)_{q}-R⁵; R⁵ = pyridine, q = 1)

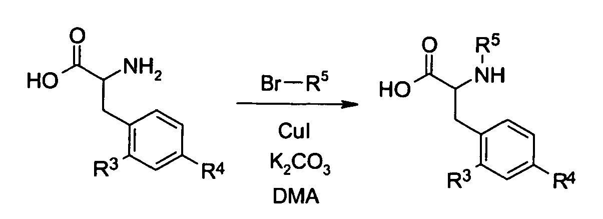

Aminopyridine derived B-C moieties can be synthesized as shown in Reaction scheme 24. Optionally substituted phenylalanine can be converted to the corresponding B-C moiety by reation with optionally substituted bromopyridines in the presence of a reagent such as copper(I) iodide and a base such as potassium carbonate in an appropriate solvent such as DMA at a suitable temperature.

### Reaction scheme 25:

### Coupling of A Moiety with B-C Moiety and Salt Formation

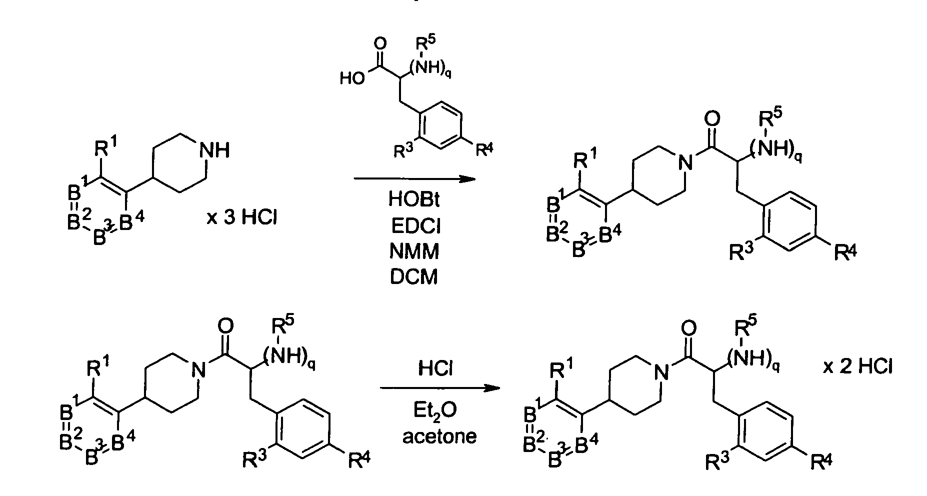

As shown in Reaction scheme 25, A moieties can be coupled with B-C moieties in the presence of EDCl/HOBt, HOAt or HATU, a base such as N-methylmorpholine (NMM) and a solvent such as dichloromethane (DCM). A suitable solvent, such as DCM, DMF, THF or a mixture of the above solvents, can be used for the coupling procedure. A suitable base includes triethylamine (TEA), diisopropylethylamine (DIEA), N-methylmorpholine (NMM), collidine or 2,6-lutidine. A base may not be needed when EDCl/HOBt is used.

Generally after the reaction is completed, the reaction mixture can be diluted with an appropriate organic solvent, such as EtOAc, DCM or Et₂O, which is then washed with aqueous solutions, such as water, HCl, NaHSO₄, bicarbonate, NaH₂PO₄, phosphate buffer (pH 7), citric acid, brine or any combination thereof. The reaction mixture can be concentrated and then be partitioned between an appropriate organic solvent and an aqueous solution. The reaction mixture can be concentrated and subjected to chromatography without aqueous workup.
The product can be transferred to a pharmaceutically acceptable salt such as a hydrochloride, using HCl in a solvent or solvent mixture such as diethyl ether/acetone.

### Analytical LC-MS

The compounds of the present invention according to formula (I) were analyzed via analytical LC-MS. The conditions used in the analysis are summarized below.

### Analytical conditions summary:

LC10Advp-Pump (Shimadzu) with SPD-M10Avp UV/Vis diode array detector and QP2010 MS-detector in ESI+ modus with UV-detection at 214, 254 and 275 nm,
Column: Waters XTerra MS C18, 3.5 µm, 2.1 * 100 mm,
linear gradient with acetonitrile in water (0.1 % HCOOH)
Flow rate of 0,4 ml/min;

| | |
|---|---|
| Mobile Phase A: | water (0.1 % HCOOH) |
| Mobile Phase B: | acetonitrile (0.1% HCOOH) |

### Gradient A:

linear gradient from 1% to 95% acetonitrile in water (0.1% HCOOH)

| | |
|---|---|
| 0.00 min | 1% B |
| 5.00 min | 95 % B |
| 5.10 min | 99 % B |
| 6.40 min | 99 % B |
| 6.50 min | 1 % B |
| 8.00 min | Pump STOP |

### Gradient B:

linear gradient from 5% to 95% acetonitrile in water (0.1% HCOOH)

| | |
|---|---|
| 0.00 min | 5% B |
| 10.00 min | 95% B |
| 10.10 min | 99% B |
| 11.40 min | 99% B |
| 11.50 min | 1% B |
| 13.00 min | Pump STOP |

### Gradient C:

start concentration 10% acetonitrile (0.1 % HCOOH)

| | |
|---|---|
| 10.00 B.Conc | 60 |
| 11.00 B.Curve | 2 |
| 12.00 B.Conc | 99 |
| 15.00 B.Conc | 99 |
| 15.20 B.Conc | 10 |
| 18.00 Pump STOP | |

### Gradient D:

start concentration 15% acetonitrile (0.1 % HCOOH)

| | |
|---|---|
| 12.00 B.Conc | 99 |
| 15.00 B.Conc | 99 |
| 15.20 B.Conc | 15 |
| 18.00 STOP 0 | |

The following tables describe detailed examples of the invention which can be prepared according to the Reaction schemes 1 to 25. These examples are, however, not construed to limit the scope of the invention in any manner.

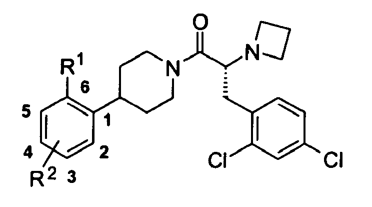

**Table 1:**

| | | | | HPLC | | MS | |
|---|---|---|---|---|---|---|---|
| No. | salt | R¹ | R² | t_{R} (min) | method | MW (calc.) free base | [M+H]⁺ (found) |
| 1 | 2 x HCl | 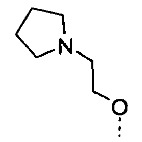 | 4-Cl | 3.78 | B | 563 | 283* |

| | | | | | | | |
|---|---|---|---|---|---|---|---|
| * [M+2H]²⁺ | | | | | | | |

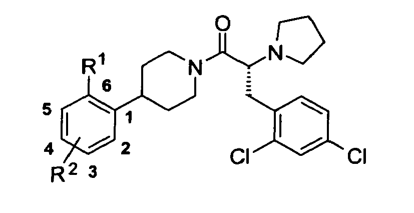

**Table 2:**

| | | | | HPLC | | MS | |
|---|---|---|---|---|---|---|---|
| No. | salt | R¹ | R² | t_{R} (min) | method | MW (calc.) free base | [M+H]⁺ (found) |
| 2 | 2 x HCl | 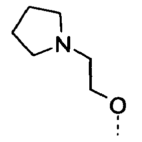 | 4-Cl | 3.90 | B | 577 | 578 |
| 3 | 2 x HCl | 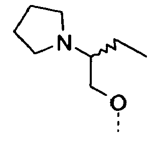 | 4-Cl | 3.81 | B | 605 | 304* |
| 4 | 2 x HCl | 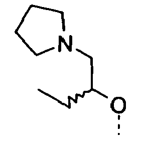 | 4-Cl | 3.93 | B | 605 | 304* |
| 5 | 2 x HCl | 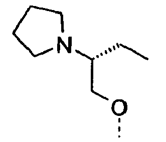 | 4-Cl | 4.22 | B | 605 | 606 |
| 6 | 2 x HCl | 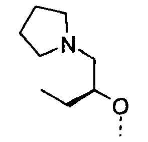 | 4-Cl | 4.27 | B | 605 | 606 |
| 7 | 2 x HCl | 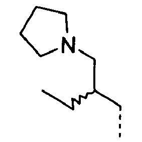 | 4-Cl | 3.98 | B | 603 | 604 |

| | | | | | | | |
|---|---|---|---|---|---|---|---|
| * [M+2H]²⁺ | | | | | | | |

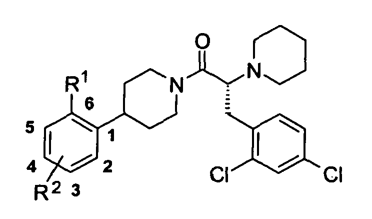

**Table 3:**

| | | | | HPLC | | MS | |
|---|---|---|---|---|---|---|---|
| No. | salt | R¹ | R² | t_{R} (min) | method | MW (calc.) free base | [M+H]⁺ (found) |
| 8 | 2 x HCl | 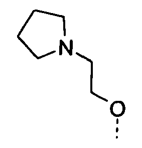 | H | 3.63 | B | 557 | 558 |
| 9 | 2 x HCl | 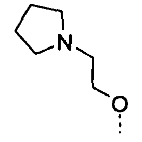 | 4-F | 3.83 | B | 575 | 576 |
| 10 | 2 x HCl | 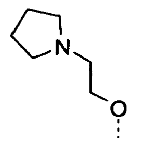 | 4-Cl | 3.98 | B | 591 | 592 |
| 11 | 2 x HCl | 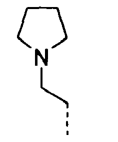 | H | 3.53 | B | 541 | 542 |
| 12 | 2 x HCl | 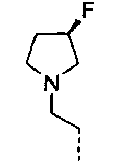 | H | 3.62 | B | 559 | 560 |
| 13 | 2 x HCl | 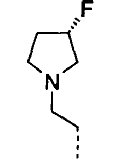 | H | 3.48 | B | 559 | 560 |
| 14 | 2 x HCl | 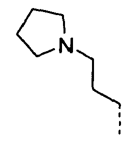 | H | 3.69 | B | 555 | 556 |
| 15 | 2 x HCl | 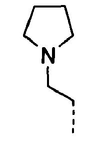 | 3-F | 3.64 | B | 559 | 560 |
| 16 | 2 x HCl | 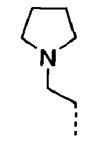 | 4-F | 3.68 | B | 559 | 560 |

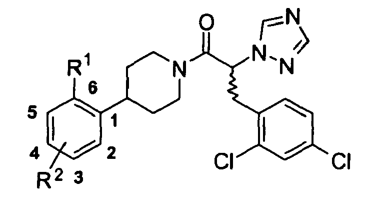

**Table 4:**

| | | | | HPLC | | MS | |
|---|---|---|---|---|---|---|---|
| No. | salt | R¹ | R² | t_{R} (min) | method | MW (calc.) free base | [M+H]⁺ (found) |
| 17 | 2 x HCl | 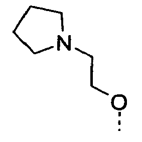 | 4-Cl | 4.76 | B | 575 | 576 |
| 18 | 2 x HCl | 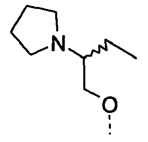 | 4-Cl | 5.16 | B | 603 | 604 |
| 19 | 2 x HCl | 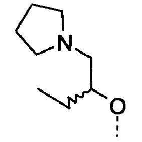 | 4-Cl | 5.16 | B | 603 | 604 |
| 20 | 2 x HCl | 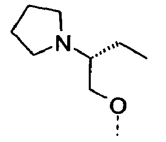 | 4-Cl | 7.49 | C | 603 | 604 |
| 21 | 2 x HCl | 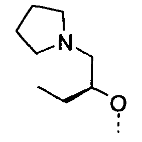 | 4-Cl | 7.41 | C | 603 | 604 |
| 22 | 2 x HCl | 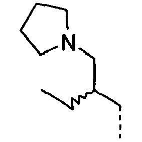 | 4-Cl | 3.30 | A | 601 | 602 |

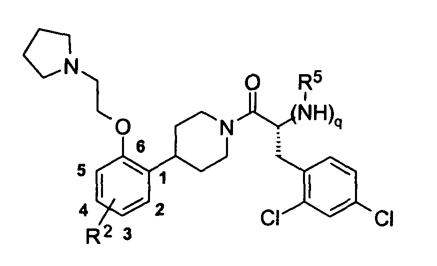

**Table 5:**

| | | | | HPLC | | MS | |
|---|---|---|---|---|---|---|---|
| No. | salt | (NH)_{q}-R⁵ | R² | t_{R} (min) | method | MW (calc.) free base | [M+H]⁺ (found) |
| 23 | - | 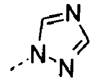 | 4-Cl | 6.42 | C | 575 | 576 |
| 24 | - | 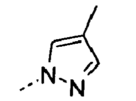 | 4-Cl | 7.54 | C | 588 | 589 |
| 25 | 2 x HCl | 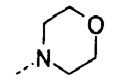 | 4-Cl | 3.95 | B | 593 | 594 |
| 26 | 2 x HCOOH | 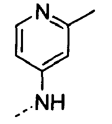 | 4-Cl | 5.25 | C | 614 | 615 |
| 27 | 2 x HCOOH | 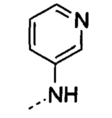 | 4-Cl | 5.52 | C | 600 | 601 |
| 28 | 2 x HCOOH | 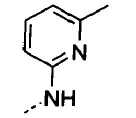 | 4-Cl | 5.51 | C | 614 | 615 |

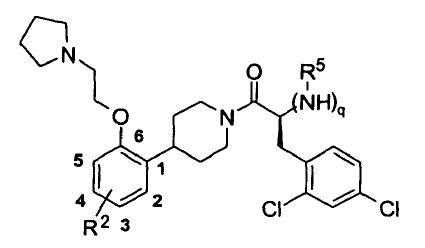

**Table 6:**

| | | | | HPLC | | MS | |
|---|---|---|---|---|---|---|---|
| No. | salt | (NH)_{q}-R⁵ | R² | t_{R} (min) | method | MW (calc.) free base | [M+H]⁺ (found) |
| 29 | - | 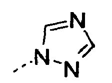 | 4-Cl | 6.47 | C | 575 | 576 |
| 30 | - | 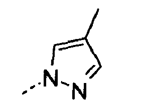 | 4-Cl | 7.56 | C | 588 | 589 |

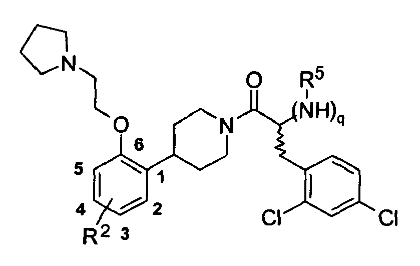

**Table 7:**

| | | | | HPLC | | MS | |
|---|---|---|---|---|---|---|---|
| No. | salt | (NH)_{q}-R⁵ | R² | t_{R} (min) | method | MW (calc.) free base | [M+H]⁺ (found) |
| 31 | 1 x HCOOH | 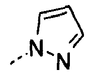 | 4-Cl | 6.05 | D | 574 | 575 |
| 32 | 1 x HCOOH | 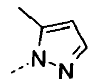 | 4-Cl | 7.60 | C | 588 | 589 |
| 33 | 1 x HCOOH | 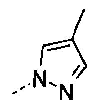 | 4-Cl | 7.74 | C | 588 | 589 |
| 34 | 1 x HCOOH | 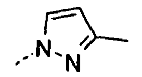 | 4-Cl | 7.83 | C | 588 | 589 |
| 35 | 1 x HCOOH | 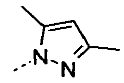 | 4-Cl | 7.86 | C | 602 | 603 |
| 36 | 1 x HCOOH | 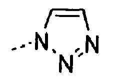 | 4-Cl | 7.24 | C | 575 | 576 |
| 37 | 1 x HCOOH | 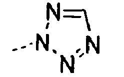 | 4-Cl | 7.35 | C | 576 | 577 |

The following examples are provided to illustrate the invention and are not limiting the scope of the invention in any manner.

### Synthesis of B-C Moieties:

### B-C Moiety 1:

### Intermediate A1):

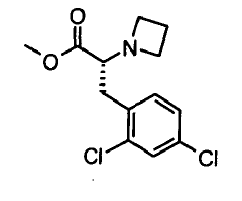

To a mixture of D-2,4-dichlorophenylalanine (700 mg), 1,3-dibromopropane (364 µl) and 1 M aqueous NaOH (3 ml) in ethanol (7 ml) was added dropwise 1 M aqueous NaOH (7.5 ml) under reflux condition. The reaction mixture was kept under reflux for 18 h. 1,3-Dibromopropane (152 µl) was added and the reaction mixture was kept under reflux for another 6 h. The reaction mixture was cooled down and acidified to pH 1-2 with 5 N HCl. The solvents were evaporated and the yellow residue was dried over Sicapent under vacuum overnight. The residue was extracted with hot ethanol (40 ml) and filtered. The filtrate was evaporated to dryness and the solid was dissolved in hot ethanol (50 ml), and ether (100 ml) was added. A white solid was starting to precipitate. The suspension was put in the fridge overnight and filtered. The filtrate was evaporated to dryness.

The crude product was dissolved in a mixture of methanol (9 ml) and toluene (35 ml) and trimethylsilyldiazomethane (2 M in hexane, 2.54 ml) was added dropwise at 0°C. The reaction mixture was stirred at room temperature for 1 h. The solvent was evaporated and the crude product was subjected to column chromatography.

### B-C Moiety 1:

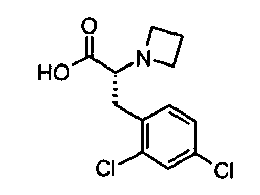

Intermediate A1) (50 mg) was dissolved in MeOH (0.1 ml) and THF (0.5 ml) at 0°C. A 0.5 M solution of lithium hydroxide monohydrate in water (0.4 ml) was added and the mixture was stirred at room temperature overnight. Volatiles were removed under reduced pressure. The residue was taken up with water and extracted twice each with diethyl ether and DCM. The aqueous phase was acidified to get pH 7/8 with 1 N aq. HCl (0.2 ml) and extracted four times each with diethyl ether and DCM. The aqueous phase was evaporated under reduced pressure and the white residue was dried under high vaccum over Sicapent to give a white powder.

### B-C Moiety 2:

### Intermediate A2):

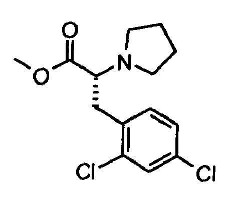

To a mixture of D-2,4-dichlorophenylalanine (700 mg), 1,3-dibromobutane (424 µl) and 1 M aqueous NaOH (3 ml) in ethanol (7 ml) was added dropwise 1 M aqueous NaOH (7.5 ml) under reflux condition. The reaction mixture was kept under reflux for 18 h. The reaction mixture was cooled down and acidified to pH 1-2 with 5 N HCl. The solvents were evaporated and the yellow residue was dried over Sicapent under vacuum overnight. The residue was extracted with hot ethanol (40 ml) and filtered. The filtrate was evaporated to dryness and the solid was dissolved in hot ethanol (15 ml) and ether (200 ml) was added. A beige solid was starting to precipitate. The suspension was put in the fridge overnight and filtered. The filtrate was evaporated to dryness.
The crude product was dissolved in a mixture of methanol (6.3 ml) and toluene (25 ml) and trimethylsilyldiazomethane (2 M in hexane, 2.53 ml) was added dropwise at 0°C. The reaction mixture was stirred at room temperature for 1 h. The solvent was evaporated and the crude product was subjected to column chromatography.

### B-C Moiety 2:

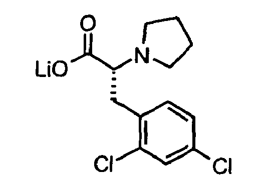

Intermediate A2) (228 mg) was dissolved in MeOH (0.15 ml) and THF (2.5 ml) at 0°C. A 0.5 M solution of lithium hydroxide monohydrate in water (1.66 ml) was added and the mixture was stirred at room temperature overnight. Volatiles were removed under reduced pressure and the residue was dried under high vaccum over Sicapent to give a white powder.

### B-C Moiety 3:

### Intermediate A3):

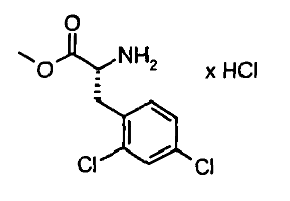

To a suspension of D-2,4-dichlorophenylalanine (10.00 g) in methanol (100 ml) was added dropwise thionylchloride (9.39 ml). During the course of the addition a clear solution was formed and the reaction started to reflux. The reaction mixture was kept under reflux for 2 h. After cooling to room temperature the mixture was evaporated to dryness at 40°C. The crude product was triturated in diethyl ether, and the insoluble compound was filtered off, washed with diethyl ether, and finally dried *in vacuo* at room temperature over P₂O₅ overnight. The product was obtained in form of colorless needles.

### Intermediate B3):

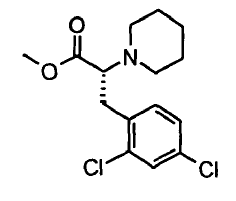

A solution of intermediate A3) (1.50 g) in water (30 ml) was brought at 0°C to pH 9.0 with aqueous ammonia (0.5 ml). The mixture was extracted twice with diethyl ether (375 ml and 150 ml, resp.). The combined organic layer was washed with brine (30 ml), dried over anhydrous Na₂SO₄, filtered and concentrated *in vacuo*.
A solution of the free base in THF (61 ml) and solid NaBH₄ (1.59 g) were slowly added simultaneously to a solution of 50% aqueous glutaraldehyde (14.4 ml) and 20% H₂SO₄ (5.75 ml) in THF (30 ml) over a period of 15 min at room temperature. The reaction mixture was stirred for additional 15 min and then was brought to pH 9.0 with diluted aqueous KOH. The resulting suspension was extracted with ethyl acetate (250 ml), and the organic layer was washed with brine (100 ml), dried over anhydrous Na₂SO₄, filtered and evaporated to yield a yellow oil. The crude compound was purified by column chromatography.

### B-C Moiety 3:

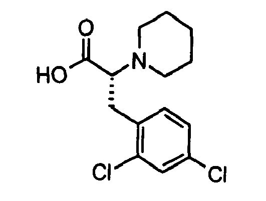

Intermediate B3) (300 mg) was dissolved in MeOH (0.9 ml) and THF (2.0 ml) at 0°C. A 0.5 M solution of lithium hydroxide monohydrate in water (2.3 ml) was added and the mixture was stirred at room temperature overnight. Volatiles were removed under reduced pressure and water (60 ml) was added to the residue. The aqueous phase was extracted with diethyl ether (45 ml). Then the aqueous phase was acidified to pH 7 with 1 M aqueous HCl and concentrated to a volume of 20 ml. A precipitate started to crush out. The aqueous phase was extracted with DCM (60 ml). The organic layer was directly filtered and the white solid was dried over Sicapent under vacuum. The aqueous phase was extracted with DCM (10 x 60 ml). The combined organic layer was evaporated under vacuum to yield a second batch of product as white solid.

### B-C Moiety 4:

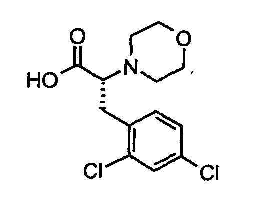

A mixture of D-2,4-dichlorophenylalanine (100 mg) and bis-(2-bromoethyl) ether (64 µl) were dissolved in 1 M NaOH (0.4 ml) and ethanol (1 ml). The mixture was heated to reflux. 1 M NaOH (1.1 ml) was added dropwise over a period of 6 h. The reaction was kept under reflux for another 12 h. Bis-(2-bromoethyl) ether (27 µl) and 1 M NaOH (300 µl) were added and the reaction was kept under reflux for 10 h. The reaction mixture was cooled down to room temperature and acidified to pH 1-2 with 6 M HCl (250 µl). The solvents were evaporated and the yellow residue was dried over Sicapent under vacuum. The crude product was extracted with hot ethanol, concentrated to half of the volume and diethyl ether was added to the solution. The solution was filtered, the filtrate concentrated and warm ethanol (1 ml) was added to dissolve it back followed by diethyl ether (13 ml). A semi solid formed on the wall of the flask. Ethanol (200 µl) was added and a white solid started to crush out. The flask was kept in the fridge for 16 h. The white precipitate was filtered off and rinsed with diethyl ether to yield a white powder.

### B-C Moiety 5:

### Intermediate A5):

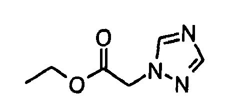

A solution of bromoethylacetate (1.0 ml) in DMF (11 ml) was added dropwise to a suspension of 1,2,4-triazole sodium derivative (816 mg) in DMF (17 ml) at -10°C. The resulting suspension was warmed to room temperature and stirred for 20 h. The reaction mixture was then concentrated *in vacuo*. The residue was partitioned between water (45 ml) and DCM (2 x 100 ml). The organic layer was dried over Na₂SO₄, filtered, and concentrated to give a pale yellow oil.

### Intermediate B5):

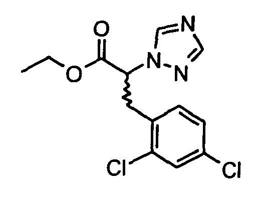

To a suspension of NaH (60% dispersion) (247 mg) in DMF (8 ml) was added intermediate A5) (800 mg) in DMF (8 ml). The reaction mixture was stirred at room temperature for 2 h and cooled down with an iced-bath. 2,4-α-Trichlorotoluene (715 µl) was slowly added and the reaction mixture was stirred at 0°C for 1 h, at room temperature for 4 h, and under reflux over night. The reaction mixture was diluted with EtOAc (100 ml) and washed with brine (50 ml), 1N aq. HCl sol. (50 ml), sat. NaHCO₃ (50 ml), water (50 ml), and brine (50 ml). The organic layer was dried over Na₂SO₄ and evaporated *in vacuo*. The crude product was purified by column chromatography and then by preparative HPLC-MS.

### B-C Moiety 5:

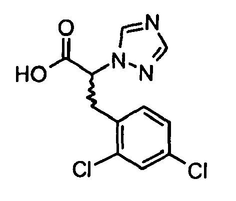

Intermediate B5) (200 mg) was dissolved in THF (2.5 ml) and 0.5N aq. LiOH (2.5 ml) was added. The reaction mixture was stirred at room temperature over night and acidified to pH = 3 with 1 N aq. HCl. EtOAc (100 ml) and water (50 ml) were added and the aq. layer was extracted with DCM (2 x 100 ml). The org. layer was dried over Na₂SO₄ and evaporated *in vacuo* to give a white solid.

### B-C Moiety 6:

### Intermediate A6):

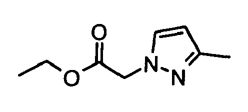

A solution of 3-methylpyrazole (720 µl) in THF (10 ml) was added dropwise to a suspension of NaH (60% dispersion) (432 mg) in THF (35 ml) at 0°C. The resulting suspension was warmed to room temperature, stirred for 1 h and then cooled to 0°C. A solution of bromoethylacetate (1.0 ml) in THF (5 ml) was added and the resulting suspension was stirred at room temperature over night. The reaction mixture was then concentrated *in vacuo* and the residue was partitioned between water (45 ml) and DCM (2 x 100 ml). The org. layer was dried over Na₂SO₄, filtered, and concentrated to give a pale yellow oil. The crude compound was purified by column chromatography.

### Intermediate B6):

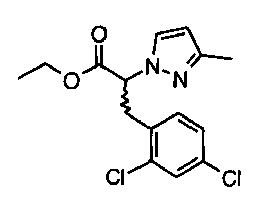

To a suspension of NaH (60% dispersion) (142 mg) in THF (5 ml) was added intermediate A6) (500 mg) in THF (5 ml). The reaction mixture was cooled down with an ice-bath and stirred for 5 min. 2,4-α-Trichlorotoluene (410 µl) was slowly added and the reaction mixture was stirred at 0°C for 1 h, at 5°C for 1 h, and under reflux for 18 h. The reaction mixture was diluted with EtOAc (100 ml) and washed with 1N aq. HCl sol. (50 ml), sat. NaHCO₃ (50 ml), water (50 ml), and brine (50 ml). The organic layer was dried over Na₂SO₄ and evaporated *in vacuo*. The crude product was purified by column chromatography.

### B-C Moiety 6:

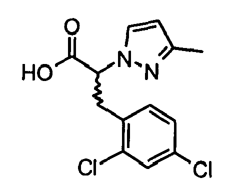

Intermediate B6) (300 mg) was dissolved in THF (4 ml) and 0.5 N aq. LiOH (4 ml) was added. The reaction mixture was stirred at room temperature for 1 h and acidified to pH = 1 with 1N aq. HCl. The two obtained layers were separated. EtOAc (100 ml) was added to the org. layer, the solution was dried over Na₂SO₄ and evaporated *in vacuo*. The obtained compound was dried under vacuum.

### B-C Moiety 7:

### Intermediate A7):

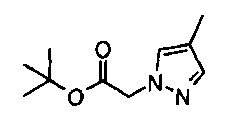

A solution of 4-methylpyrazole (723 µl) in THF (10 ml) was added dropwise to a suspension of NaH (60% dispersion) (432 mg) in THF (35 ml) at 0°C. The resulting suspension was warmed to RT, stirred for 1 h and then cooled to 0°C. A solution of bromo-t-butyl-acetate (1.3 ml) in THF (5 ml) was added. The resulting suspension was stirred at room temperature over night. The reaction mixture was then concentrated *in vacuo*. The residue was partitioned between water (45 ml) and DCM (2 x 100 ml). The organic layer was dried over MgSO₄, filtered, and concentrated to give a pale yellow oil. The crude compound was used as such for the next step.

### Intermediate B7):

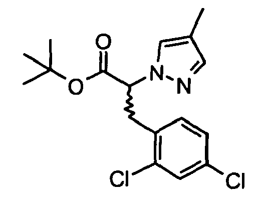

To a suspension of NaH (60% dispersion) (192 mg) in THF (10 ml) was added intermediate A7) (800 mg) in THF (5 ml) at 0°C and the reaction mixture was stirred for 5 min. 2,4-α-Trichlorotoluene (564 µl) was slowly added and the reaction mixture was stirred at 0°C for 2 h and under reflux for 1 h. The reaction mixture was diluted with EtOAc (100 ml) and washed with 1N aq. HCl sol. (50 ml), sat. NaHCO₃ (50 ml), water (50 ml), and brine (50 ml). The organic layer was dried over Na₂SO₄ and evaporated *in vacuo*. The crude product was purified by column chromatography.

### B-C Moiety 7:

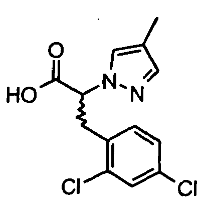

Intermediate B7) (1.4 g) was dissolved in THF (10 ml) and 0.5N aq. LiOH (10 ml) was added. The reaction mixture was stirred at 60°C overnight and acidified to pH = 1 with 1N aq. HCl. The two obtained layers were separated. EtOAc (100 ml) was added to the organic layer and the aqueous layer was extracted with EtOAc (3 x 50 ml). The combined organic layers were dried over Na₂SO₄ and evaporated *in vacuo*. The obtained compound was dried under vacuum.

### B-C Moiety 8:

### Intermediate A8):

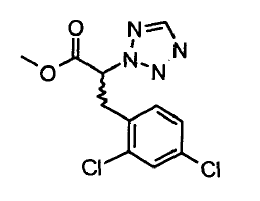

To a suspension of NaH (60% dispersion) (154 mg) in THF (5 ml) was added methyl 2-(2-tetrazolyl) acetate (500 mg) in THF (5 ml). The reaction mixture was stirred at room temperature for 30 min. The mixture was cooled down with an ice-bath and 2,4-α-trichlorotoluene (440 µl) was slowly added. The reaction mixture was kept under reflux for 24 h. After cooling to room temperature, the reaction mixture was diluted with EtOAc (100 ml) and washed with 1 N aq. HCl sol. (50 ml), sat. NaHCO₃ (50 ml), water (50 ml), and brine (50 ml). The organic layer was dried over Na₂SO₄ and evaporated *in vacuo*. The crude product was purified by column chromatography and then by preparative HPLC-MS.

### B-C Moiety 8:

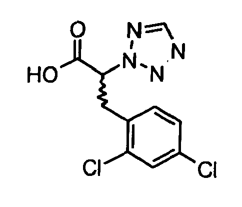

Intermediate A8) (40 mg) was dissolved in THF (1 ml) and 0.5N aq. LiOH (1 ml) was added. The reaction mixture was stirred at room temperature for 2 h and acidified to pH = 1 with 1 N aq. HCl. The two obtained layers were separated. EtOAc (30 ml) was added to the organic layer, the solution was dried over Na₂SO₄ and evaporated *in vacuo*. The obtained compound was dried under vacuum.

### B-C Moiety 9:

### Intermediate A9):

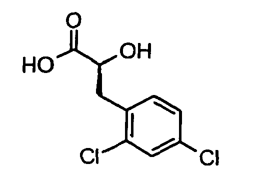

L-2,4-dichlorophenylalanine (2.00 g) was dissolved in 0.5 N aq. hydrochloric acid (42 ml). NaNO₂ (1.77 g) was added slowly at -5°C. The reaction mixture was allowed to stir at 0°C for 2 hours then at room temperature for 2 hours. The excess of nitrogen oxide was removed by bubbling argon through the solution for 30 min. Water (80 ml) was added and the mixture was extracted two times with diethyl ether (200 ml and 100 ml, resp.). The combined organic layer was dried over Na₂SO₄, filtered and evaporated to yield a pale yellow oil.

### Intermediate B9):

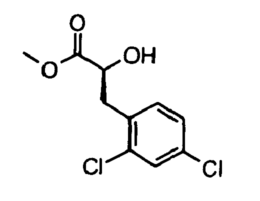

Thionylchloride (1.63 ml) was slowly added to methanol (25 ml) while cooling in an ice bath. After 5 min, intermediate A9) (1.75 g) in MeOH (10 ml) was added and the reaction mixture was stirred at room temperature under argon atmosphere for 15 h. The solvent was evaporated and remaining thionyl chloride was removed by codistillation with toluene (2 x 30 ml). The oily, yellow residue was taken up in DCM (250 ml) and washed with 1N NaHCO₃ (125 ml), water (80 ml) and brine (50 ml). The organic phase was dried over Na₂SO₄, filtered and evaporated. The crude compound was purified by column chromatography to yield the product as yellow oil.

### Intermediate C9):

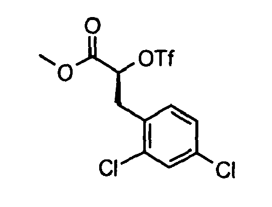

A solution of intermediate B9) (1.00 g) and 2,6-lutidine (794 µl) in dry DCM (17 ml) was cooled to -15°C. Trifluoromethanesulfonic anhydride (1.08 ml) was added dropwise and the reaction was stirred at -10°C for 3 h. The reaction mixture was diluted with diethyl ether (30 ml) and the organic phase was extracted with 1N aq. HCl (80 ml) and brine (170 ml). The organic phase was dried over Na₂SO₄, filtered and evaporated.

### Intermediate D9):

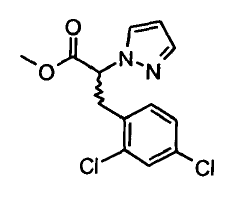

A solution of pyrazole (99 mg) in dry THF (7 ml) was treated with sodium hydride (60% in mineral oil, 217 mg) at 0°C. The mixture was stirred at room temperature until gas evolution ceased (30 min). Then a solution of the intermediate C9) (600 mg) in dry THF (4 ml) was dropwise added to the white suspension of the pyrazole sodium salt at 0°C within 2 min. The resulting, clear yellow solution was stirred at room temperature overnight. THF was evaporated, the residue taken up with ethyl acetate (100 ml) and washed with water (25 ml). The aqueous phase was extracted again with ethyl acetate (100 ml). The aqueous layer was acidified with 1N aqueous HCl to pH 6 and the product was extracted with ethyl acetate (2 x 100 ml). The combined organic layer was washed with brine (25 ml), dried over Na₂SO₄, filtered and evaporated to yield a yellow solid.

### B-C Moiety 9:

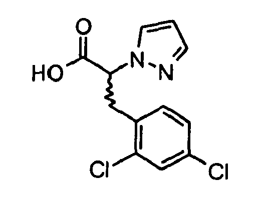

Intermediate D9) (238 mg) was dissolved in THF (3 ml) and 0.5N aq. LiOH (1.9 ml) was added followed by methanol (50 µl). The reaction mixture was stirred at room temperature overnight. THF and methanol were evaporated under reduced pressure. The residue was taken up with water (4 ml) and the aqueous phase was washed with diethyl ether (10 ml) and DCM (10 ml). The aqueous phase was acidified to pH 2 with 5N aqueous HCl and extracted with DCM (20 ml). The organic layer was dried over Na₂SO₄, filtered and evaporated under reduced pressure to yield the product as yellow solid.

### B-C Moiety 10:

### B-C Moiety 10:

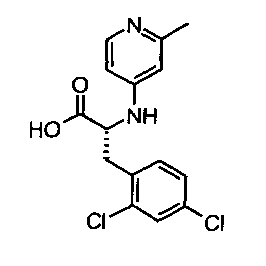

(R)-2-Amino-3-(2,4-dichloro-phenyl)-propionic acid (700 mg), K₂CO₃ (621 mg), and Cul (57 mg) were dissolved in DMA (10 ml). 4-Bromo-2-methylpyridine (312 µl) was added and the mixture was stirred at 115°C for 6 h. The solvent was evaporated *in vacuo* and to the obtained residue were added MeCN (40 ml) and water (6 ml). The mixture was filtrated and the solution was evaporated *in vacuo*. The crude product was purified by preparative HPLC-MS.

### B-C Moiety 11:

### B-C Moiety 11:

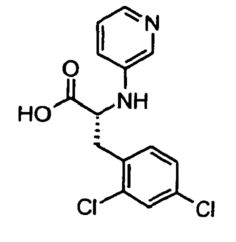

(R)-2-Amino-3-(2,4-dichloro-phenyl)-propionic acid (500 mg), K₂CO₃ (700 mg), and Cul (50 mg) were dissolved in DMA (5 ml). 3-Bromopyridine (440 µl) was added and the mixture was stirred at 110°C for 4 h. The solvent was evaporated *in vacuo* and to the obtained residue were added MeCN (30 ml) and water (8 ml). The mixture was filtrated and the solution was evaporated *in vacuo*. The crude product was purified by preparative HPLC-MS.

### Synthesis of Example 1:

### Intermediate 1a):

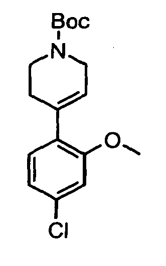

2-Bromo-5-chloroanisole (5.54 g), 1-(2(H)-pyridine-carboxylic acid-3,6-dihydro-4-(4,4,5,5-tetramethyl-1,3,2-dioxaborolan-2-yl)-tert.-butyl ester (7.73 g), dichloro(1,1'-bis(diphenyl-phosphino)-ferrocene)palladium(II) DCM adduct (1.22 g) and K₂CO₃ (10.36 g) were dissolved in degassed DMF in a dry apparatus under argon and the mixture was degassed again by evacuation followed by refilling with argon. The resulting suspension was heated in an oil bath at 85°C overnight. The mixture was cooled, filtered through Celite and evaporated to dryness. The crude product was purified by flash chromatography to yield a clear yellowish oil.

### Intermediate 1b):

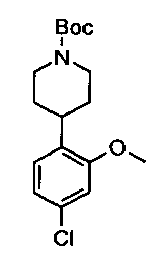

Intermediate 1a) (2.18 g) was dissolved in EtOH (80 ml) and AcOH (80 ml) under argon. Platinum(IV) oxide (0.23 g) was added and the reaction mixture was placed under an H₂ atmosphere using a balloon. The reaction mixture was then stirred at room temperature for 120 min. The reaction mixture was filtered through Celite and evaporated to dryness *in vacuo*. The residue was coevaporated with toluene (3 x 40 ml). The crude product was purified by flash chromatography to yield a clear colorless oil.

### Intermediate 1c):

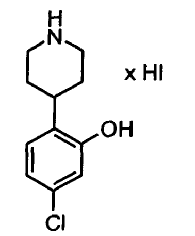

To a solution of intermediate 1b) (1.56 g) in AcOH (6.5 ml) was added hydroiodic acid (5.2 ml of a 57 wt.% aq. solution) and the mixture was heated under reflux (oil bath at 140°C) in an argon atmosphere for 2 h. The reaction mixture was cooled to room temperature and then evaporated to dryness *in vacuo*. The residue was coevaporated with toluene (3 x 30 ml). The crude product was triturated in Et₂O (40 ml), the insoluble compound was filtered off and washed with Et₂O (10 ml). Finally, the product was dried *in vacuo* over P₂O₅ at room temperature overnight to yield a white solid.

### Intermediate 1d):

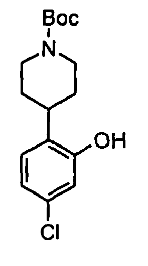

To a solution of intermediate 1c) (1.55 g) in DMF (10 ml) was added DIEA (0.88 ml) followed by di-tert.-butyl-dicarbonate (1.01 g). The reaction mixture was stirred at room temperature for 4 h. The mixture was evaporated *in vacuo* to dryness and partitioned between 0.5 M HCl (50 ml) and EtOAc (100 ml). The organic layer was washed with water (25 ml) and brine (30 ml). The organic layer was dried with MgSO₄ and evaporated in *vacuo* to dryness to yield a yellowish solid. The solid residue was triturated in EtOAc (1 ml) and Et₂O (10 ml), and left in the fridge overnight to complete crystallization of the product. The precipitate was then filtered off, washed with cold Et₂O (1 ml), and finally dried *in vacuo* at room temperature over P₂O₅ overnight. The product was obtained in form of a white solid.

### Intermediate 1e):

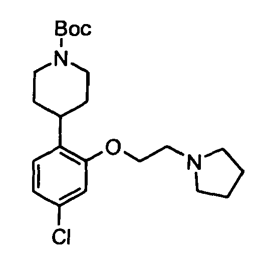

To a solution of intermediate 1d) (887 mg) in DMF (15 ml) was added 1-(3-chloroethyl)pyrrolidine hydrochloride (605 mg) and Cs₂CO₃ (3243 mg). The reaction was stirred at room temperature for 18 h. An additional amount of 1-(3-chloroethyl)pyrrolidine hydrochloride (483 mg) and Cs₂CO₃ (926 mg) were added and stirring at room temperature was continued for 6 h. The reaction mixture was evaporated at 40°C *in vacuo* to dryness and the residue was partitioned between EtOAc and water. The aqueous layer was extracted with EtOAc. The combined organic layer was washed with water and brine. The organic layer was dried over Na₂SO₄ and evaporated *in vacuo* to dryness. The crude product was purified using flash chromatography.

### Intermediate 1f):

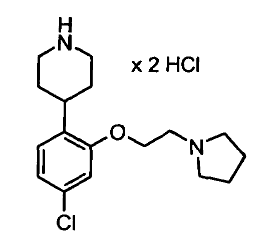

To Boc-protected intermediate 1e) (1170 mg) in dioxane (5 ml) was added hydrogen chloride, 4.0 M sol. in 1,4-dioxane (20 ml) and the solution was stirred at room temperature for 2 h. The solvent was removed under reduced pressure. The residue was triturated in acetone and Et₂O, filtered off, and washed with Et₂O. Finally, it was dried *in vacuo* at room temperature over P₂O₅ overnight to yield a white solid.

### Example 1:

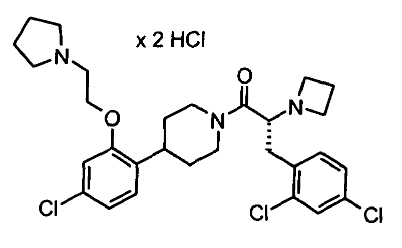

To a solution of B-C moiety 1 (148 mg, 0.430 mmol) in dry DMF (8.0 ml) was added HOBt (72 mg), EDCI (90 mg) and NMM (189 µl). The reaction mixture was stirred for 5 min and then intermediate 1f) (179 mg) was added at 0°C. The reaction stirred at room temperature overnight. The reaction mixture was diluted with ethyl acetate (40 ml) and successively washed with sat. NaHCO₃ (20 ml), followed by water (20 ml) and brine (20 ml). The organic phase was dried over Na₂SO₄, filtered and evaporated. The crude product was purified by column chromatography. The product was dissolved in ethyl acetate (50 µl) and then cooled down to 0°C prior to addition of 1 M HCl in diethyl ether (25 µl) to give a white solid. Diethyl ether (1 ml) was added; the white solid was triturated, filtered under argon atmosphere and dried over Sicapent under vacuum to yield an off-white solid.

### Synthesis of Example 3:

### Intermediate 3a):

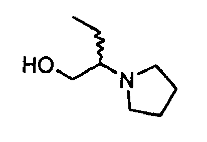

To a solution of 2-amino-1-butanol (6.33 ml) and 1,4-dibromobutane (7.91 ml) in CH₃CN (70 ml) was added K₂CO₃ (18.52 g) and the resulting suspension was stirred at reflux temperature for 22 h. The reaction mixture was evaporated in vacuo and the residue was partitioned between EtOAc and water. The organic layer was washed with water and brine. The aqueous layers were extracted with EtOAc. The combined organic layer was dried over Na₂SO₄ and evaporated in vacuo to dryness. The crude product was purified by Kugelrohr-distillation (15 mbar, 110-140°C) to yield a clear colorless oil.

### Intermediate 3b):

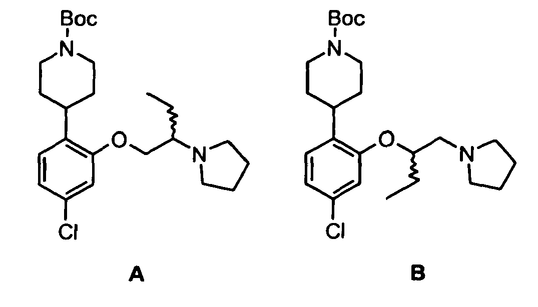

A solution of intermediate 1d) (1.09 g), intermediate 3a) (1.00 g) and triphenylphosphine (1.83 g) in dry THF (30 ml) under argon atmosphere, was cooled in an ice/H₂O bath. Then DEAD (ca. 40% in toluene, 3.20 ml) was added dropwise, at a rate to keep the temperature below 5°C. After stirring for another 10 min, the cooling bath was removed and the mixture was stirred at room temperature overnight. The reaction mixture was evaporated to dryness in vacuo at 40°C. The two regioisomeric products were separated by flash chromatography.

### Intermediate 3c):

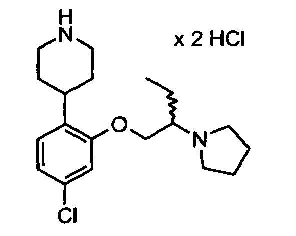

To intermediate 3b) product A (1.07 g) in dioxane (4 ml) was added hydrogen chloride, 4.0 M sol. in 1,4-dioxane (15 ml) and the solution was stirred for 2 h at room temperature. The solvent was removed under reduced pressure and the residue was triturated with acetone and diethyl ether. The product was filtered off and washed with acetone/diethyl ether. The product was obtained as an off-white solid.

### Example 3:

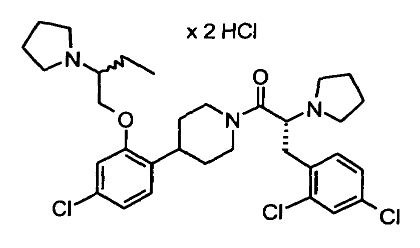

To a suspension of B-C moiety 2 (20 mg) in DMF (2 ml) was added HOBt (10 mg) and EDCI (13 mg). The reaction mixture was stirred at room temperature for 15 min and then intermediate 3c) (25 mg) and NMM (27 µl) were added and the reaction mixture was stirred at room temperature overnight. The reaction mixture was evaporated *in vacuo*, diluted with EtOAc, washed with sat. Na₂CO₃, H₂O and brine. The aqueous layers were extracted with EtOAc. The combined organic layer was dried over Na₂SO₄, filtered and evaporated *in vacuo* to dryness. The residue was purified by flash column chromatography. The residue was dissolved in CH₂Cl₂, acidified with HCl in Et₂O 1 M (69 µl) and evaporated in vacuo. The residue was dissolved in CH₂Cl₂ and the salt was precipitated by addition of Et₂O. The precipitate was filtered off, washed with Et₂O and dried in vacuo at 40°C for 2 hours. The product was obtained as white solid.

### Synthesis of Example 4:

### Intermediate 4a):

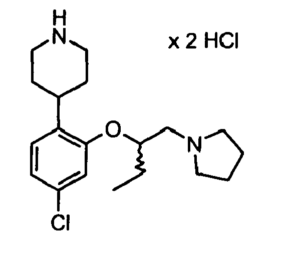

To intermediate 3b) product B (370 mg) in dioxane (1 ml) was added hydrogen chloride, 4.0 M sol. in 1,4-dioxane (5 ml) and the solution was stirred at room temperature for 2 h. The solvent was removed under reduced pressure and the residue was triturated with acetone and diethyl ether. The product was filtered off and washed with acetone/diethyl ether. The product was obtained as an off-white solid.

### Example 4:

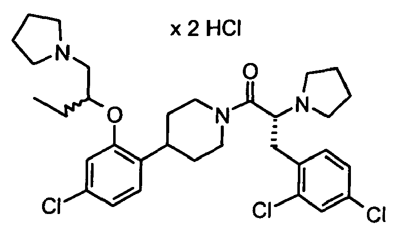

To a suspension of B-C moiety 2 (20 mg) in DMF (2 ml) was added HOBt (10 mg) and EDCI (13 mg). The reaction mixture was stirred at room temperature for 15 min and then intermediate 4a) (25 mg) and NMM (27 µl) were added and the reaction mixture was stirred at room temperature overnight. The reaction mixture was evaporated *in vacuo*, diluted with EtOAc, washed with sat. Na₂CO₃, H₂O and brine. The aqueous layers were extracted with EtOAc. The combined organic layer was dried over Na₂SO₄, filtered and evaporated *in vacuo* to dryness. The residue was purified by flash column chromatography. The residue was dissolved in CH₂Cl₂, acidified with HCl in Et₂O 1 M (76 µl) and evaporated *in vacuo*. The residue was dissolved in CH₂Cl₂ and the salt was precipitated by addition of Et₂O. The precipitate was filtered off, washed with Et₂O and dried *in vacuo* at 40°C for 2 hours. The product was obtained as white solid.

### Synthesis of Example 8:

### Intermediate 8a):

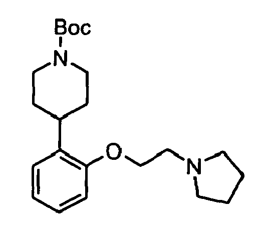

To a solution of 1-Boc-4-(2-hydroxy-phenyl)-piperidine (789 mg) in DMF (15 ml) was added 1-(2-chloroethyl)pyrrolidine hydrochloride (605 mg) and Cs₂CO₃ (3243 mg). The reaction was stirred at room temperature for 18 h. An additional amount of 1-(2-chloroethyl)pyrrolidine hydrochloride (483 mg) and Cs₂CO₃ (926 mg) was added and stirring at room temperature was continued for another 6 h. The reaction mixture was evaporated at 50°C *in vacuo* to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated *in vacuo* to dryness. The residue was finally dried under high vacuum at room temperature overnight.

### Intermediate 8b):

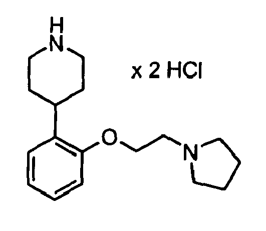

To Boc-protected intermediate 8a) (1002 mg) in methanol (5 ml) was added hydrogen chloride, 4.0 M sol. in 1,4-dioxane (25 ml) and the solution was stirred at room temperature for 2 h. The solvent was removed under reduced pressure. The residue was triturated in acetone (30 ml), filtered off, and washed with acetone (2 x 5 ml). Finally, it was dried *in vacuo* at room temperature over P₂O₅ overnight to yield a white solid.

### Example 8:

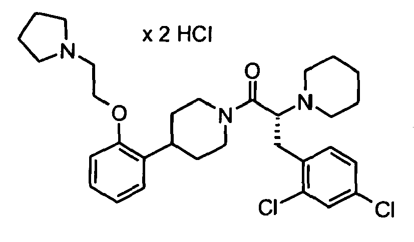

To suspension of B-C moiety 3 (40 mg) in DCM (2.0 ml) was added HOBt (20mg), EDCI (25 mg) and NMM (53 µl). The reaction mixture was stirred for 5 min and then intermediate 8b) (42 mg) was added at 0°C. The reaction mixture was mostly a white suspension. DMF (0.6 ml) was added to bring most of the reagents in solution. The reaction stirred at room temperature for 6 h. The reaction mixture was filtered and the filtrate was diluted with ethyl acetate (20 ml) and successively washed with water (6 ml), followed by sat. NaHCO₃ (15 ml) and brine (10 ml). The organic phase was dried over Na₂SO₄, filtered and the solvent was removed under reduced pressure. The crude product was purified by column chromatography. The purified product was dissolved in EtOAc (300 µl) and then 1 M HCl in diethyl ether (300 µl) was added. A white solid crushed out. Diethyl ether (1.5 ml) was added; the white solid was triturated and filtered to give a white powder that was dried over Sicapent under vaccum.

### Synthesis of Example 12:

### Intermediate 12a):

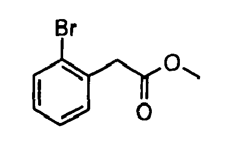

(2-Bromo-phenyl)-acetic acid (5.38 g) was dissolved in methanol (20.26 ml). Then concentrated sulfuric acid (0.27 ml) was added, and the reaction mixture was heated under reflux overnight (oil bath temperature 85°C) with exclusion of humidity by means of a drying tube (blue silica gel). The reaction mixture was evaporated *in vacuo* at 40°C and the colorless oily residue was poured into ice-water (50 ml). The resulting white emulsion was extracted with Et₂O (75 ml), and the organic phase was washed with sat. Na₂CO₃ (3 x 20 ml), H₂O (15 ml), and brine (15 ml). The organic phase was dried with MgSO₄ and evaporated *in vacuo* to yield a colorless clear oil.

### Intermediate 12b):

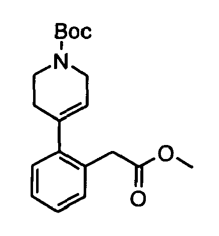

Intermediate 12a) (4.00 g), N-Boc-1,2,3,6-tetrahydropyridine-4-boronic acid pinacol ester (5.40 g), potassium carbonate (7.24 g), and dichloro(1,1'-bis(diphenyl-phosphino)-ferrocene)palladium(II) DCM adduct (860 mg) were dissolved in DMF (150 ml) in a dry apparatus under argon and the mixture was degassed by bubbling with argon for 30 min. The orange suspension was then heated under argon in an oil bath at 85°C overnight. The reaction mixture was filtered through Celite and evaporated to dryness *in vacuo*. The residue was triturated in DCM (50 ml) and the insoluble parts were filtered off. The filtrate was concentrated and subjected to flash chromatography. The product was obtained as clear yellow oil.

### Intermediate 12c):

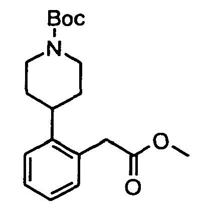

Intermediate 12b) (1.99 g) was dissolve in EtOH (30 ml) and AcOH (30 ml) and platinum(IV) oxide (400 mg) was added. The reaction mixture was evacuated three times and purged with hydrogen. The reaction mixture was then stirred at room temperature for 2 h. The reaction mixture was filtered and evaporated to dryness *in vacuo*. The residue was coevaporated with toluene (3 x 75 ml) and was finally dried under high vacuum at room temperature overnight.

### Intermediate 12d):

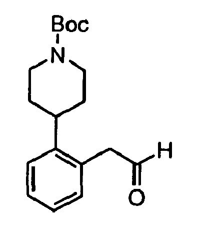

Intermediate 12c) (2.85 g) was dissolved in dry diethyl ether (30 ml) under inert atmosphere and cooled to -72°C. At this temperature diisobutylaluminum hydride, 1.0 M in hexane (12.8 ml) was added dropwise in the course of 30 min. The reaction mixture was stirred at -72°C for 2 h. Methanol (173 µl) was added and the mixture was warmed up to 0°C. Water (1.5 ml) was added and the mixture was filtered through a bed of sodium sulfate. After washing twice with diethyl ether (30 ml each) the combined organic filtrate was concentrated *in vacuo*. The crude product was purified by flash chromatography to yield a colorless oil.

### Intermediate 12e):

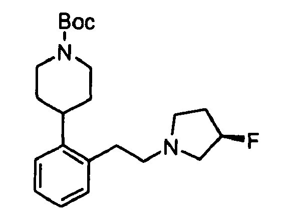

To a solution of the intermediate 12d) (121 mg) and (R)-3-fluoropyrrolidine hydrochloride (50 mg) in dichloroethane (5 ml), DIEA (139 µl) was added followed by sodium triacetoxyborohydride (119 mg). The reaction mixture was then stirred for 4 h at room temperature. The mixture was diluted with EtOAc (70 ml) and washed two times with sat. NaHCO₃ (25 ml each), water and brine (25 ml each). The organic phase was dried over Na₂SO₄ and concentrated. The crude product was purified using flash chromatography.

### Intermediate 12f):

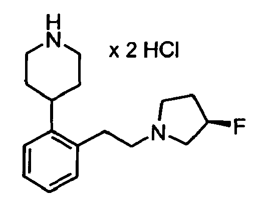

To intermediate 12e) (102 mg) in dioxane (5 ml) and methanol (1 ml) was added hydrogen chloride, 4.0 M sol. in 1,4-dioxane (5 ml) and the solution was stirred for 30 min at room temperature. The solvent was removed under reduced pressure, the residue was triturated with acetone (5 ml) and diethyl ether (25 ml) and the product was filtered off.

### Example 12:

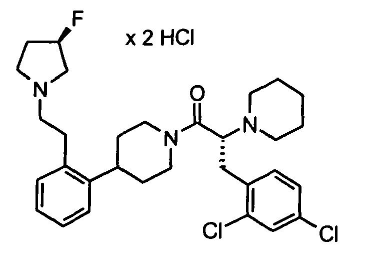

To suspension of B-C moiety 3 (34 mg) in DCM (2 ml) was added HOBt (17 mg), EDCI (22 mg) and NMM (45 µl). The reaction mixture was stirred for 5 min and then intermediate 12f) (36 mg) was added at 0°C. The reaction mixture was mostly a white suspension. The reaction was stirred at room temperature overnight. The reaction mixture was filtered and the filtrate was diluted with ethyl acetate (20 ml) and successively washed with water (6 ml), followed by sat. NaHCO₃ (15 ml) and brine (10 ml). The organic phase was dried over Na₂SO₄, filtered and the solvent was removed under reduced pressure. The crude product was purified by column chromatography. The purified product was dissolved in EtOAc (300 µl) and then 1 M HCl in diethyl ether (400 µl) was added. A white solid crushed out. Diethyl ether (1.5 ml) was added; the white solid was triturated, filtered under argon atmosphere and dried over Sicapent under vaccum.

### Synthesis of Example 15:

### Intermediate 15a):

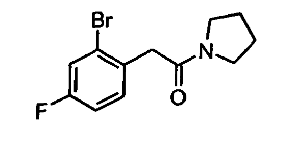

2-Bromo-4-fluorophenylacetic acid (2330 mg), EDCI (2109 mg) and DMAP (100 mg) were dissolved in DCM (100 ml). Pyrrolidine (918 µl) was added and the reaction mixture was stirred overnight. The reaction mixture was poured into water (100 ml) and the organic layer was separated. The aqueous layer was extracted twice with DCM. The combined organics were washed three times with 0.5 N HCl (30 ml each), three times with 1 M sodium hydroxide solution and brine, dried over Na₂SO₄ and the solvent was removed under reduced pressure. The crude product was purified by flash chromatography.

### Intermediate 15b):

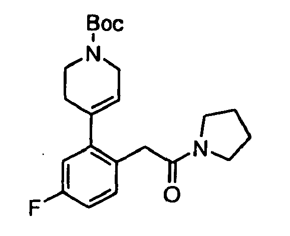

Intermediate 15a) (1692 mg), N-Boc-1,2,3,6-tetrahydropyridine-4-boronic acid pinacol ester (1920 mg), potassium carbonate (2450 mg), and dichloro(1,1'-bis(diphenyl-phosphino)-ferrocene)palladium(II) DCM adduct (286 mg) were dissolved in DMF (70 ml) in a dry apparatus under argon and the mixture was degassed by bubbling with argon for 30 min. The orange suspension was then heated under argon in an oil bath at 85°C overnight. The reaction mixture was filtered through Celite and evaporated to dryness *in vacuo*. The residue was triturated in DCM (50 ml) and the insoluble parts were filtered off. The filtrate was concentrated and subjected to flash chromatography.

### Intermediate 15c):

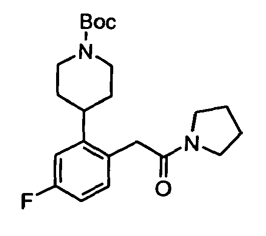

Intermediate 15b) (2266 mg) was dissolved in EtOH (50 ml) and AcOH (50 ml) and platinum(IV) oxide (132 mg) was added. The reaction mixture was evacuated three times and purged with hydrogen. The reaction mixture was then stirred at room temperature for 2 h. The reaction mixture was filtered and evaporated to dryness *in vacuo*. The residue was coevaporated with toluene (3 x 75 ml) and was finally dried under high vacuum at room temperature overnight. The crude product was purified by flash chromatography to yield a white solid.

### Intermediate 15d):

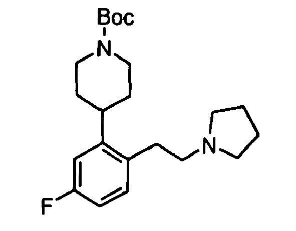

Intermediate 15c) (1392 mg) in diethyl ether (20 ml) was slowly added to a mixture of lithium aluminum hydride (203 mg) and diethyl ether (30 ml) at 0°C. After addition the reaction mixture was stirred at 0°C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The product was purified by flash chromatography.

### Intermediate 15e):

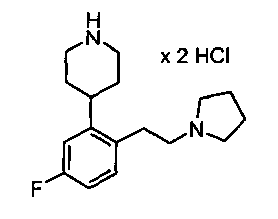

To intermediate 15d) (373 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (10 ml) and diethyl ether (50 ml) and the product was filtered off.

### Example 15:

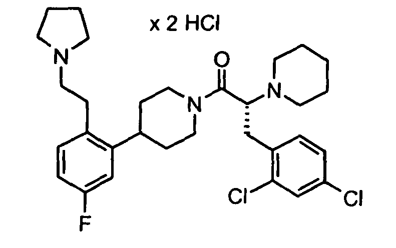

To a suspension of B-C moiety 3 (33 mg) in DCM (1.5 ml) was added HOBt (15 mg), EDCI (19 mg) and NMM (39 µl). The reaction mixture was stirred for 5 min and then intermediate 15e) (31 mg) was added at 0°C. The reaction mixture was mostly a white suspension. The reaction was stirred at room temperature overnight. The reaction mixture was filtered and the filtrate was diluted with ethyl acetate (20 ml) and successively washed with water (6 ml), followed by sat. NaHCO₃ (15 ml) and brine (10 ml). The organic phase was dried over Na₂SO₄, filtered and the solvent was removed under reduced pressure. The crude product was purified by column chromatography. The purified product was dissolved in EtOAc (300 µl) and then 1 M HCl in diethyl ether (300 µl) was added. A white solid crushed out. Diethyl ether (1.5 ml) was added; the white solid was triturated and filtered under argon atmosphere to yield a white powder that was dried over Sicapent under vaccum.

### Synthesis of Example 20:

### Intermediate 20a):

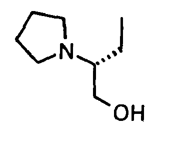

To a solution of (R)-(-)-2-amino-1-butanol (5.00 g) and 1,4-dibromobutane (6.61 ml) in CH₃CN (60 ml) was added K₂CO₃ (15.48 g) and the resulting suspension was stirred at reflux temperature for 19 hours. The reaction mixture was evaporated in vacuo and the residue was divided between EtOAc and water. The organic layer was washed with water and brine. The aqueous layers were extracted with EtOAc. The combined organic layer was dried over Na₂SO₄ and evaporated in vacuo to dryness. The crude product was purified by Kugelrohr-distillation (15 mbar, 110-150°C) to yield a clear colorless oil.

### Intermediate 20b):

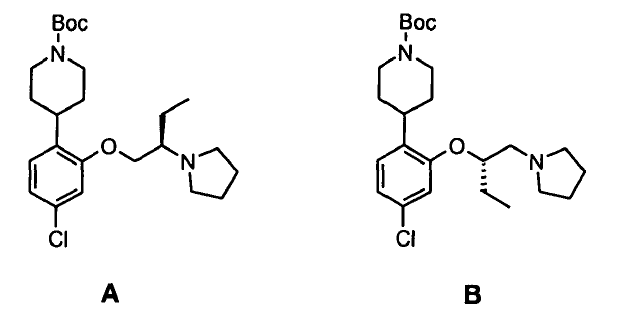

A solution of intermediate 1d) (1.09 g), intermediate 20a) (1.00 g), and triphenylphosphine (1.83 g) in THF (30 ml) under argon, was cooled in ice/H₂O. DEAD (ca. 40% in toluene, 3.20 ml) was added dropwise, at a rate to keep the temperature below 5°C (ca. 15 min). After stirring for another 10 min in ice/H₂O, the cooling bath was removed and the mixture was stirred at 40°C overnight. The reaction mixture was evaporated to dryness *in vacuo* at 40°C. The two regioisomeric products A and B were separated by flash chromatography.

### Intermediate 20c):

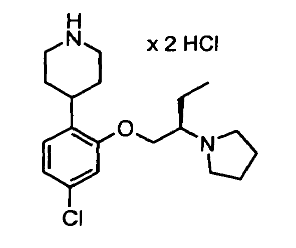

To Boc-protected intermediate 20b) (product A) (1230 mg) in dioxane (4 ml) was added hydrogen chloride, 4.0 M sol. in 1,4-dioxane (15 ml) and the solution was stirred at room temperature for 90 min. The solvent was removed under reduced pressure. The residue was triturated in a mixture of isopropanol, acetone, Et₂O and hexane, filtered off, and washed with acetone/Et₂O. Finally it was dried *in vacuo* at room temperature over P₂O₅ overnight to yield a beige solid.

### Example 20:

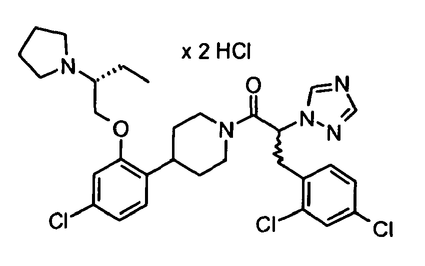

To a suspension of B-C moiety 5 (118 mg) in DMF (12 ml) was added HOBt (62 mg) and EDCI (77 mg). The reaction mixture was stirred at room temperature for 15 min and then intermediate 20c) (150 mg) and NMM (161 µl) were added and the reaction mixture was stirred at room temperature overnight. The reaction mixture was evaporated *in vacuo*, diluted with EtOAc, washed with sat. Na₂CO₃, H₂O and brine. The aqueous layers were extracted with EtOAc. The combined organic layer was dried over Na₂SO₄, filtered and evaporated *in vacuo* to dryness. The residue was purified by flash column chromatography. The residue was dissolved in CH₂Cl₂, acidified with HCl in Et₂O 1 M (266 µl) and evaporated *in vacuo*. The residue was dissolved in CH₂Cl₂ and the salt was precipitated by addition of Et₂O. The precipitate was filtered off, washed with Et₂O and dried over Sicapent for 3 h followed by drying in vacuo at 40°C for 2 hours. The product was obtained as white solid.

### Synthesis of Example 21:

### Intermediate 21a):

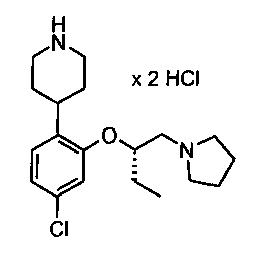

To Boc-protected intermediate 20b) (product B) (744 mg) in dioxane (2.5 ml) was added hydrogen chloride, 4.0 M sol. in 1,4-dioxane (9.0 ml) and the solution was stirred at room temperature for 90 min. The reaction mixture was evaporated to dryness in vacuo. The oily residue was triturated in i-propanol, acetone and Et₂O, the formed solid was filtered off and washed with acetone, Et₂O and i-propanol.

### Example 21:

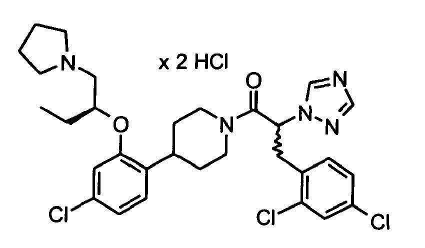

To a suspension of B-C moiety 5 (118 mg) in DMF (12 ml) was added HOBt (62 mg) and EDCI (77 mg). The reaction mixture was stirred at room temperature for 15 min and then intermediate 21a) (150 mg) and NMM (161 µl) were added and the reaction mixture was stirred at room temperature overnight. The reaction mixture was evaporated *in vacuo*, diluted with EtOAc, washed with sat. Na₂CO₃, H₂O and brine. The aqueous layers were extracted with EtOAc. The combined organic layer was dried over Na₂SO₄, filtered and evaporated *in vacuo* to dryness. The residue was purified by flash column chromatography. The residue was dissolved in CH₂Cl₂, acidified with HCl in Et₂O 1 M (258 µl) and evaporated *in vacuo*. The residue was dissolved in CH₂Cl₂ and the salt was precipitated by addition of Et₂O. The precipitate was filtered off, washed with Et₂O and dried over Sicapent for 3 h followed by drying *in vacuo* at 40°C for 2 hours. The product was obtained as beige solid.

### Synthesis of Example 22:

### Intermediate 22a):

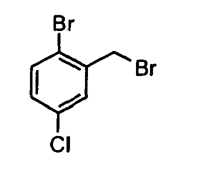

2-Bromo-5-chlorotoluene (8.26 ml) and N-bromosuccinimide (11.03 g) in carbon-tetrachloride (50 ml) were treated with a catalytic amount of benzoylperoxide (100 mg) and heated under reflux until the reaction had reached completion as monitored by TLC. The reaction mixture was then allowed to cool and filtered. The filtrate was washed twice with water and brine, dried over sodium sulfate and concentrated *in vacuo*.

### Intermediate 22b):

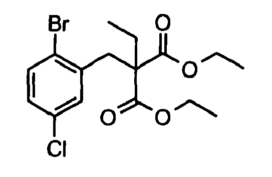

To a solution of sodium ethoxide (2.72 g) in ethanol (60 ml) was added diethyl ethylmalonate (7.87 ml) and and intermediate 22a) (11.38 g) was slowly added and the reaction mixture was kept under reflux overnight. The reaction mixture was evaporated in *vacuo* and the residue was partitioned between diethyl ether and water and the aqueous layer extracted two times with diethyl ether. The combined organic layer was washed twice with water and brine. The combined organic layer was dried over Na₂SO₄ and evaporated *in vacuo* to dryness. The crude product was purified column chromatography.

### Intermediate 22c):

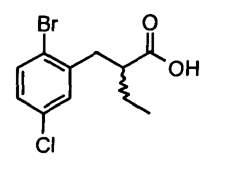

Intermediate 22b) (21.11 g) was heated under reflux in 1.8 M KOH in H₂O/EtOH (150 ml) for 5 h. After evaporation of the ethanol an additional amount of KOH (54 g) was added to the residue, and the reaction mixture was stirred for 2 h at 100°C. The reaction mixture was diluted with 200 ml of H₂O, extracted with Et₂O and the organic layer was washed with H₂O. The combined aqueous layer was cooled with ice/H₂O and acidified with 50 % H₂SO₄ to pH 1. The precipitate was extracted twice with Et₂O (200 ml each) and the organic layers were washed with water and brine. The combined organic layers were dried over Na₂SO₄ and evaporated *in vacuo* to dryness. The residue was triturated in hexane and less Et₂O, then filtered and washed with hexane and less Et₂O. The solid residue was decarboxylated by heating at 200°C. The development of CO₂ ceased after 20 min and the melt was cooled to room temperature.

### Intermediate 22d):

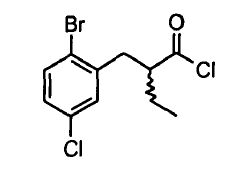

Intermediate 22c) (2.85 g) was dissolved in dry DCM (85 ml) and cooled to 0°C in an ice-water bath. Oxalyl chloride (0.91 ml) in DCM (15 ml) was added dropwise followed by the addition of 1-2 drops of DMF. This mixture was stirred at 0°C for 1.5 h and at room temperature for 2 h and then concentrated. The product was dried under reduced pressure.

### Intermediate 22e):

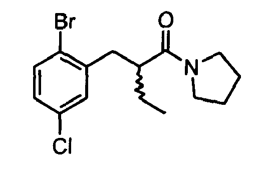

To a solution of intermediate 22d) (2.95 g) in DCM (70 ml) at 0°C was added pyrrolidine (2.00 ml) dropwise and the reaction mixture was stirred at 0°C for 2 h. The reaction mixture was diluted with DCM (100 ml) and washed twice each with 1 M HCl, 1 M NaOH and once with brine. The organic phase was dried over sodium sulfate and the solvent was distilled off.

### Intermediate 22f):

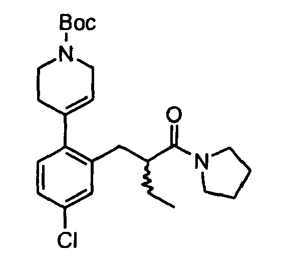

Intermediate 22e) (3111 mg), N-Boc-1,2,3,6-tetrahydropyridine-4-boronic acid pinacol ester (2931 mg), potassium carbonate (3744 mg) and dichloro(1,1'-bis(diphenyl-phosphino)-ferrocene)palladium(II) DCM adduct (441 mg) were dissolved in DMF (100 ml) in a dry apparatus under argon and the mixture was degassed by bubbling with argon for 30 min. The orange suspension was then heated under argon in an oil bath at 85°C for 1 day to give a dark purple suspension. The reaction mixture was filtered through Celite and evaporated to dryness *in vacuo*. The residue was dissolved in ethyl acetate (100 ml) and the insoluble parts were filtered off. The filtrate was extracted twice with water and with brine, dried over sodium sulfate and the solvent was evaporated. The crude product was purified by column chromatography.

### Intermediate 22g):

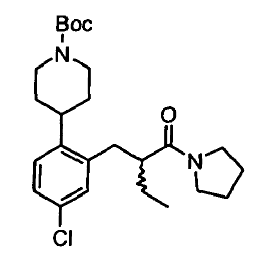

Intermediate 22f) (3103 mg) was dissolved in EtOH (50 ml) and AcOH (50 ml) and platinum(IV) oxide (157 mg) was added. The reaction mixture was evacuated three times and purged with hydrogen. The reaction mixture was then stirred at room temperature for 2 h. The reaction mixture was filtered and evaporated to dryness *in vacuo*. The residue was coevaporated with toluene (3 x 75 ml) and was finally dried under high vacuum at room temperature overnight.

### Intermediate 22h):

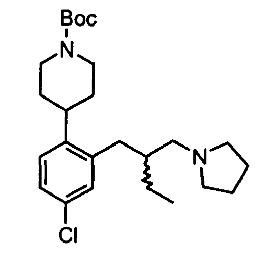

Intermediate 22g) (2225 mg) in diethyl ether (20 ml) was slowly added to a mixture of lithium aluminum hydride (286 mg) and diethyl ether (30 ml) at 0°C. After addition, the reaction mixture was stirred at 0°C for 2.5 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The crude product was purified by flash chromatography to yield a colorless oil.

### Intermediate 22i):

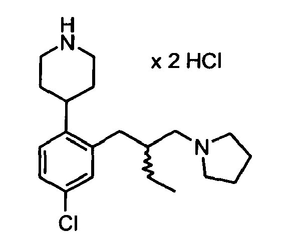

To intermediate 22h) (326 mg) in dioxane (5 ml) and methanol (1 ml) was added hydrogen chloride, 4.0 M sol. in 1,4-dioxane (5 ml) and the solution was stirred for 30 min at room temperature. The solvent was removed under reduced pressure, the residue was triturated with acetone (5 ml) and diethyl ether (50 ml) and the product was filtered off. The product was obtained as off-white solid.

### Example 22:

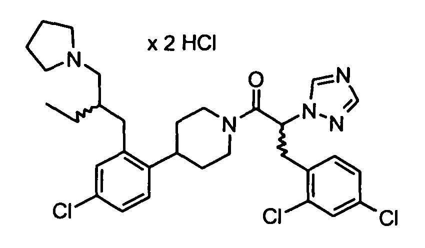

To a solution of B-C moiety 5 (38 mg) in dry DMF (2.5 ml) was added HOBt (23 mg), EDCI (28 mg) and NMM (65 µl). The reaction mixture was stirred for 5 min and then intermediate 22i) (60 mg) was added at 0°C. The reaction stirred at room temperature overnight. DMF was evaporated and the residue was diluted with ethyl acetate (25 ml) and successively washed with sat. NaHCO₃ (20 ml), followed by water (20 ml) and brine (20 ml). The organic phase was dried over Na₂SO₄, filtered and the solvent was removed under reduced pressure. The crude product was purified by column chromatography. The product was dissolved in ethyl acetate (50 µl) and then cooled down to 0°C prior to addition of 1M HCl in diethyl ether (150 µl) to give a white solid. Diethyl ether (1.0 ml) was added; the white solid was triturated, filtered under argon atmosphere and dried over Sicapent under vacuum to yield the product as off-white solid.

### Synthesis of Examples 24 and 30:

### Example 24 and 30:

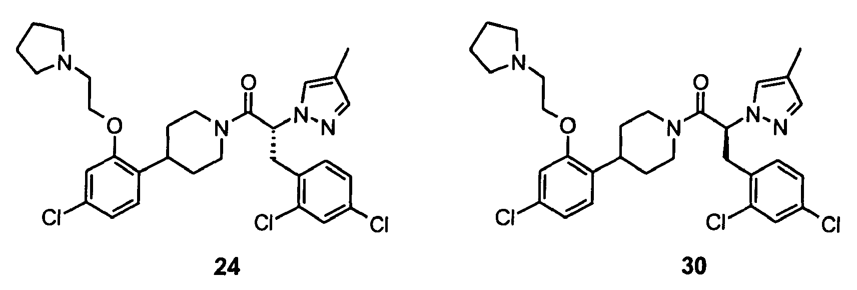

B-C moiety 7 (135 mg) was dissolved in dry DMF (8 ml). HOBt (96 mg), EDCI (120 mg), and DIEA (118 µl) were added. The reaction mixture was stirred at room temperature for 5 min and intermediate 1f) (153 mg) was added. The reaction mixture was stirred at room temperature for 1 h, diluted with EtOAc (100 ml) and washed with brine (50 ml), 0.5N aq. citric acid sol. (50 ml), sat. NaHCO₃ (50 ml) and brine (50 ml). The organic layer was dried over Na₂SO₄ and evaporated *in vacuo*. The crude product was purified by preparative HPLC-MS to afford the compound as racemate. The two title compounds were obtained by purification with chiral HPLC-MS.

### Synthesis of Example 26:

### Example 26:

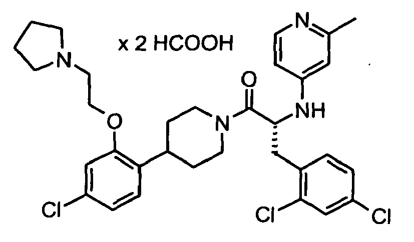

B-C moiety 10 (80 mg) was dissolved in dry DMF (1 ml). HATU (105 mg) and DIEA (131 µl) were added. The reaction mixture was stirred at room temperature for 5 min and intermediate 1f) (95 mg) in DMF (1 ml) and DIEA (50 µl) was added. The reaction mixture was stirred at room temperature for 24 h, diluted with EtOAc (50 ml) and washed with brine (50 ml), 0.5N aq. citric acid sol. (50 ml), sat. NaHCO₃ (50 ml) and brine (50 ml). The organic layer was dried over Na₂SO₄ and evaporated *in vacuo*. The crude product was purified by preparative HPLC-MS.

### Synthesis of Example 27:

### Example 27:

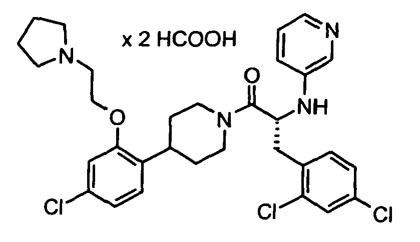

B-C moiety 11 (65 mg) was dissolved in dry DMF (1 ml). HOBt (45 mg), EDCI (57 mg), and NMM (35 µl) were added. The reaction mixture was stirred at room temperature for 5 min and intermediate 1f) (76 mg) in DMF (1 ml) was added. The reaction mixture was stirred at room temperature for 24 h. An additional amount of NMM (50 µl) was added and stirring continued at room temperature overnight. An additional amount of HOBt (50 mg) was added and stirring continued at room temperature for 6 h. The reaction mixture was diluted with EtOAc (50 ml) and washed with brine (50 ml), 0.5N aq. citric acid sol. (50 ml), saturated NaHCO₃ (50 ml) and brine (50 ml). The organic layer was dried over Na₂SO₄ and evaporated *in vacuo*. The crude product was purified by preparative HPLC-MS.

### Synthesis of Example 31:

### Example 31:

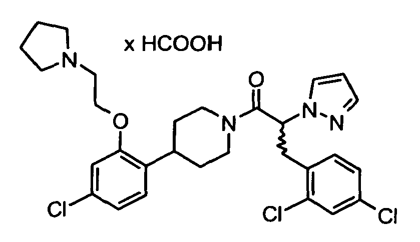

B-C moiety 9 (61 mg) was dissolved in dry DCM (2 ml) and dry DMF (0.5 ml). HOBt (33 mg), EDCI (41 mg), and NMM (85 µl) were added. The reaction mixture was stirred at room temperature for 5 min and intermediate 1f) (74 mg) was added at 0°C. The reaction mixture was stirred at room temperature overnight, diluted with EtOAc (20 ml) and washed with water (10 ml), saturated NaHCO₃ (10 ml) and brine (10 ml). The organic layer was dried over Na₂SO₄ and evaporated *in vacuo*. The crude product was purified by column chromatography followed by preparative HPLC-MS.

### Synthesis of Example 37:

### Example 37:

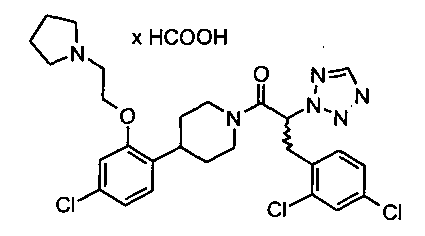

B-C Moiety 8 (30 mg) was dissolved in dry DMF (1 ml). HOBt (21 mg), EDCI (27 mg), and DIEA (26 µl) were added. The reaction mixture was stirred at room temperature for 5 min and intermediate 1f) (40 mg) was added. The reaction mixture was stirred at room temperature for 20 h, diluted with EtOAc (50 ml) and washed with brine (25 ml), 0.5N aq. citric acid sol. (25 ml), sat. NaHCO₃ (25 ml) and brine (25 ml). The organic layer was dried over Na₂SO₄ and evaporated *in vacuo*. The crude product was purified by preparative HPLC-MS.

### Biological Assays

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4°C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have IC₅₀ values less than 2 µM.

**Table 8: Biological data for selected examples of the invention**

| Example | hMC-4R binding assay IC₅₀/nM | hMC-4R functional assay EC₅₀/nM | % activation functional assay |
|---|---|---|---|
| SHU-9119 | 1.9 | - | 7 |
| NDP-α-MSH | 1.1 | 3.4 | 100 |
| 3 | 2.1 | - | 0 |
| 4 | 2.4 | - | 0 |
| 10 | 62 | - | 14 |
| 15 | 180 | - | 0 |
| 22 | 17 | - | 0 |
| 23 | 7.7 | - | 0 |
| 25 | 100 | - | 0 |
| 27 | 5.9 | - | 0 |
| 34 | 220 | - | 0 |

### C. In Vivo Food Intake Models

### 1. Spontaneous Feeding Paradigm

Food intake in rats is measured after i.p., s.c. or p.o. administration of the test compound (see e.g. A.S. Chen et al. Transgenic Res 2000 Apr ;9(2):145-154).

### 2. Model of LPS-Induced Anorexia and Tumor-Induced Cachexia

Prevention or amelioration of anorexia induced by either lipopolysaccharide (LPS) administration or cachexia induced by tumor growth is determined upon i.p. or p.o. administration of test compounds to rats (see e.g. D.L. Marks, N. Ling, and R.D. Cone, Cancer Res 2001 Feb 15;61(4):1432-1438).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how, and if, an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in K.E. McKenna et al, A Model For The Study of Sexual Function In Anesthetized Male And Female Rats, Am. J. Physiol. (Regulatory Integrative Comp. Physiol 30): R1276-R1285, 1991; K.E. McKenna et al, Modulation By Peripheral Serotonin of The Threshold For Sexual Reflexes In Female Rats, Pharm. Bioch. Behav., 40:151-156, 1991; and L.K. Takahashi et al, Dual Estradiol Action In The Diencephalon And The Regulation of Sociosexual Behavior In Female Golden Hamsters, Brain Res., 359: 194-207, 1985.

### Examples of a Pharmaceutical Composition

As a specific embodiment of an oral composition of a compound of the present invention, 28 mg of Example 5 is formulated with sufficient finely divided lactose to provide a total amount of 580 to 590 mg to fill a size 0 hard gelatin capsule.

As another specific embodiment of an oral composition of a compound of the present invention, 26 mg of Example 21 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound according to formula (I) 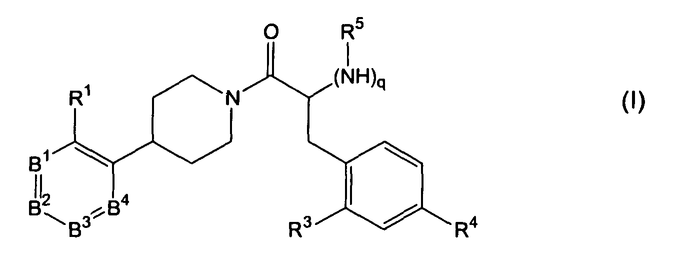 and the enantiomers, diastereomers, tautomers, solvates and pharmaceutically acceptable salts thereof,
wherein
R¹ is -N(R¹⁰)-(C(R⁶)₂)ₘ-T
-(C(R⁶)₂)ₗ-T, or
-O-(C(R⁶)₂)ₘ-T;
R⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T is NR⁷R⁸,
morpholine, 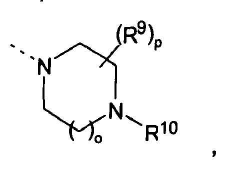 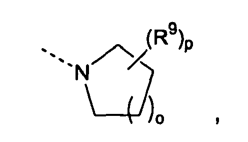 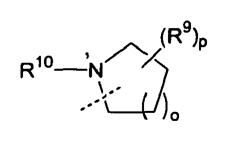 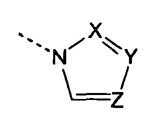 or 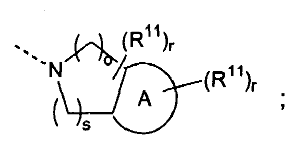
R⁷ and R⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH, and
C₁₋₆-alkylene-O-C₁₋₆-alkyl optionally substituted with 1 to 3 substituents selected from halogen, CN and OH;
R¹⁰ is H, or
C₁-C₆-alkyl;
R¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X is CH or N;
Y is CH or N;
Z is CH or N;
A is a 3-7-membered saturated, unsaturated or aromatic ring containing 0-2 nitrogen atoms;
B¹ to B⁴ are independently selected from CR² and N;
R² is independently selected from
H,
F,
Cl,
CH₃,
OCH₃, and
CF₃;
R³ is H,
Cl,
F, or
CH₃;
R⁴ is Cl or F;
R⁵ is 4 to 7-membered heterocyclyl containing 1 or 2 heteroatoms independently selected from the group consisting of NR¹², O and S; or
4 to 7-membered heteroaryl containing 1 to 4 heteroatoms independently selected from the group consisting of N, O and S;
wherein each heterocyclyl may be unsubstituted or substituted by 1 or 2 substituents selected from the group consisting of oxo group, CN, C₁₋₆-alkyl, C₃₋₆-cycloalkyl, halogen and OH, and
each heteroaryl may be unsubstituted or substituted by 1 or 2 substituents selected from the group consisting of C₁₋₆-alkyl, C₃₋₆-cycloalkyl, O-C₁₋₆-alkyl, halogen and CN;
R¹² is hydrogen,
C₁₋₆-alkyl,
C₃₋₆-cycloalkyl and
C(O)C₁₋₆-alkyl;
I is 0, 1, 2, 3, or 4;
m is 0, 1, 2, 3, or 4;
o is 0, 1, or 2;
p is 0, 1, 2, 3, or 4;
q is 0, or 1;
r is 0, 1, 2, 3, or 4 and
s is 1, or 2.

2. The compound of claim 1 according to formula (I') 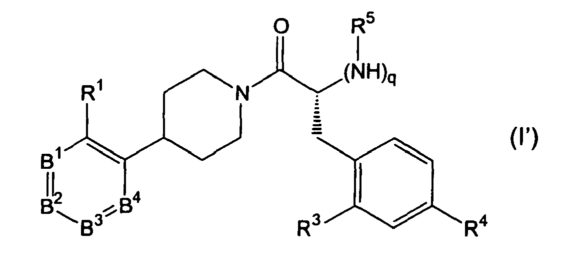 wherein B¹, B², B³, B⁴, R¹, R³, R⁴, R⁵ and q are as defined in claim 1.

3. The compound of claim 1 or 2, wherein at least one of R⁷ and R⁸ is selected from
C₂₋₆-alkenyl,
C₂₋₆-alkinyl, and
C₂₋₆-alkylene-O-C₁₋₆-alkyl.

4. The compound of any of claims 1 to 3, wherein
R² is selected from H, F, Cl and CH₃.

5. The compound of any of claims 1 to 4 wherein
I is 2 or 3, or
m is 2 or 3.

6. The compound of any of claims 1 to 5 wherein
R⁵ is 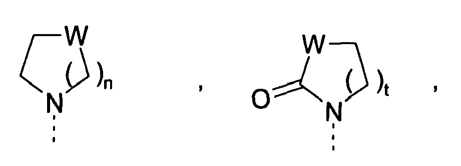 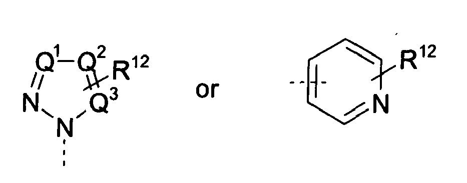 wherein
W is CH₂, NR¹², O;
Q¹, Q², Q³ are independently from each other a nitrogen atom or a carbon atom, with the proviso that at least one of Q¹, Q² and Q³ is a carbon atom;
R¹² is hydrogen,
C₁₋₆-alkyl, or
C(O)C₁₋₆-alkyl;
n is 0, 1, or 2 and
t is 1 or 2.

7. The compound of any of claims 1 to 6 wherein
R⁵ is 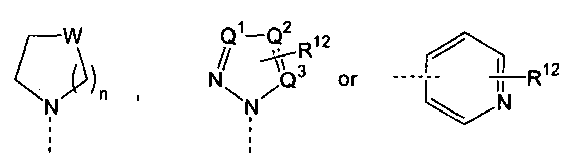 wherein
n is 0 to 2,
W is CH₂ in the case of n = 0 or 1 and
W is CH₂, NR¹² or O in the case of n = 2,
Q¹, Q², Q³ are independently from each other a nitrogen atom or a carbon atom, with the proviso that at least one of Q¹, Q² and Q³ is a carbon atom; and
R¹² is hydrogen,
C₁₋₆-alkyl, or
C(O)C₁₋₆-alkyl.

8. The compound of any of claims 1 to 7 as medicament.

9. Use of the compound of any of claims 1 to 7 for the preparation of a medicament for the treatment or prophylaxis of disorders, diseases or conditions responsive to the inactivation or activation of the melanocortin-4 receptor in a mammal.

10. Use according to claim 9 for the preparation of a medicament for the treatment or prophylaxis of cancer cachexia.

11. Use according to claim 9 for the preparation of a medicament for the treatment or prophylaxis of muscle wasting.

12. Use according to claim 9 for the preparation of a medicament for the treatment or prophylaxis of anorexia.

13. Use according to claim 9 for the preparation of a medicament for the treatment or prophylaxis of anxiety and/or depression.

14. Use according to claim 9 for the preparation of a medicament for the treatment or prophylaxis of obesity.

15. Use according to claim 9 for the preparation of a medicament for the treatment or prophylaxis of diabetes mellitus.

16. Use according to claim 9 for the preparation of a medicament for the treatment or prophylaxis of male or female sexual dysfunction.

17. Use according to claim 9 for the preparation of a medicament for the treatment or prophylaxis of erectile dysfunction.

18. A pharmaceutical composition comprising a compound of any of claims 1 to 7 and a pharmaceutically acceptable carrier.
